# EUROPEAN PATENT APPLICATION

(11) **EP 3 745 134 A1**
(43) Date of publication of application: **02.12.2020**
(21) Application number: 18901831.0
(22) Date of filing: 27.12.2018
(51) Int. Cl.: G01N 35/00, C12M 1/00, C12Q 1/6844, G01N 35/02

(54) **NUCLEIC ACID ANALYSIS DEVICE AND NUCLEIC ACID EXTRACTION DEVICE**

(30) Priority: 26.01.2018 JP 2018011794
(71) Applicant: SYSMEX CORPORATION, Kobe-shi Hyogo 651-0073 (JP)
(72) Inventor: FUTAMATSU Fumiya, Kobe-shi, Hyogo 651-0073 (JP); SAKAI Yasuhiro, Kobe-shi, Hyogo 651-0073 (JP); TANOSHIMA Eiji, Kobe-shi, Hyogo 651-0073 (JP); OZAWA Toshiyuki, Kobe-shi, Hyogo 651-2271 (JP); SUZUKI Kenichiro, Kobe-shi, Hyogo 651-0073 (JP); MURATA Chikako, Kobe-shi, Hyogo 651-0073 (JP)
(74) Representative: Hoffmann Eitle
(86) International application number: PCT/JP2018/048271
(87) International publication number: WO 2019/146373

(57) **Abstract**

Provided are a nucleic acid analysis device and a nucleic acid extraction device that allow appropriate setting of a container to the device. A nucleic acid analysis device 100 includes: a plurality of amplification container setting parts 120 each configured to have an amplification container 20 set thereto, the amplification container 20 being configured to have injected therein an extract that contains nucleic acid, the amplification container 20 storing a reagent for amplifying the nucleic acid in the extract; a display unit 402; and a controller 401 configured to cause the display unit 402 to display a screen 500 in which a location of each of the plurality of amplification container setting parts 120 is associated with relevant information regarding setting of the amplification container 20 to the amplification container setting part 120.

## Description

### TECHNICAL FIELD

The present invention relates to a nucleic acid analysis device and a nucleic acid extraction device.

### BACKGROUND ART

In recent years, in association with prevalence of genetic tests, there is a demand for a device that automatically performs processes of extraction to detection of nucleic acid.

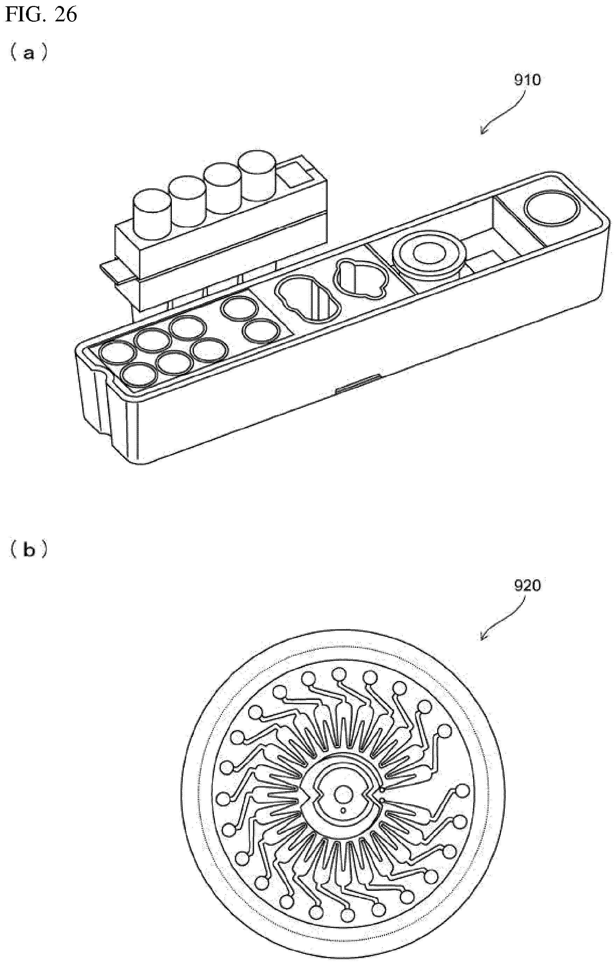

PATENT LITERATURE 1 describes a nucleic acid analysis device that amplifies and analyzes a target nucleic acid contained in a specimen. In this nucleic acid analysis device, a nucleic acid purification kit 910 shown in FIG. 26(a) and a nucleic acid analysis chip 920 shown in FIG. 26(b) are used for extraction and detection of the target nucleic acid. The nucleic acid purification kit 910 is used in order to separate nucleic acid from a sample and to purify a nucleic acid solution. The nucleic acid analysis chip 920 is used in order to amplify the target nucleic acid from the nucleic acid extracted by using the nucleic acid purification kit 910 and to detect the target nucleic acid.

### CITATION LIST

### [PATENT LITERATURE]

[PTL 1] Japanese Patent No. 5003845

### SUMMARY OF THE INVENTION

### PROBLEMS TO BE SOLVED BY THE INVENTION

A nucleic acid analysis device can be used in various diagnoses of colon cancer, leukemia, and the like. In this case, the site of nucleic acid to be analyzed is different for each diagnosis. Thus, for each diagnosis, a container that stores therein a reagent corresponding to the diagnosis is used. Thus, when an operator performs analysis of nucleic acid, the operator needs to appropriately set a container corresponding to the diagnosis, to the nucleic acid analysis device. However, the work of selecting a container corresponding to the diagnosis and then assuredly setting the container to an appropriate position in the nucleic acid analysis device is particularly difficult for an operator who is not familiar with the device. Therefore, there is a demand for a nucleic acid analysis device that enables an operator to appropriately set a container.

### SOLUTION TO THE PROBLEMS

A first aspect of the present invention relates to a nucleic acid analysis device. A nucleic acid analysis device (100) according to the present aspect includes: a plurality of amplification container setting parts (120) each configured to have an amplification container (20) set thereto, the amplification container (20) being configured to have injected therein an extract that contains nucleic acid, the amplification container (20) storing a reagent for amplifying the nucleic acid in the extract; a display unit (402); and a controller (401) configured to cause the display unit (402) to display a screen in which a location of each of the plurality of amplification container setting parts (120) is associated with relevant information regarding setting of the amplification container (20) to the amplification container setting part (120).

The relevant information denotes: information for specifying a sample to be processed by a reagent in an amplification container set to an amplification container setting part; information indicating whether or not an amplification container is set to the amplification container setting part; information regarding the type of the amplification container that should be set to the amplification container setting part; information indicating that the type of the amplification container set to the amplification container setting part is wrong; and the like.

In the nucleic acid analysis device according to the present aspect, the relevant information plays the role of a guide for an operator to set an amplification container to an amplification container setting part. Therefore, by referring to the relevant information displayed on the display unit, the operator can appropriately set an amplification container to each amplification container setting part. When the relevant information is displayed, even an operator who is not familiar with the nucleic acid analysis device can appropriately and smoothly set the amplification container.

In the nucleic acid analysis device (100) according to the present aspect, the controller (401) may be configured to cause the display unit (402) to display, as the relevant information, information that mutually links each of a plurality of samples to a location of an amplification container setting part (120) to which an amplification container (20) storing a reagent for processing the sample is set. Accordingly, by referring to the screen displayed on the display unit, the operator can easily understand the correspondence relationship between the sample to be processed and the amplification container setting part. Therefore, the operator can easily understand to which amplification container setting part the operator should set an amplification container to which an extract containing the nucleic acid extracted from each sample is to be injected. Therefore, the operator can appropriately set an amplification container appropriate for each sample, to the container setting part.

In the nucleic acid analysis device (100) according to the present aspect, the controller (401) may be configured to cause the display unit (402) to display, as the relevant information, information that links identification information of each of a plurality of samples to a location of an amplification container setting part (120) to which an amplification container (20) storing a reagent for processing the sample is set.

"Information that links" denotes color, digit, character, diagram, or the like. For example, the same color provided to a display region of identification information of a sample and to an image of a container setting part corresponding to the sample is information that links the display region and the image to each other.

In the nucleic acid analysis device (100) according to the present aspect, the controller (401) may be configured to cause the display unit (402) to display, as the relevant information, information regarding the type of the amplification container (20) that should be set to each amplification container setting part (120).

The type of the amplification container denotes, for example, a measurement item targeted by the reagent stored in the amplification container. For example, the measurement item is All-Ras, EGFR, ESR1, BRAF, leukemia, or the like. The information regarding the type of the amplification container is information that allows identification of the type of the amplification container, and is, for example: a character string or a diagram indicating the type of the amplification container; a character string or a diagram for identifying usage of the amplification container; or the like. A reagent container name is a character string indicating the type of the amplification container.

In this case, by referring to the relevant information displayed on the display unit, the operator can understand the type of the amplification container that should be set to the amplification container setting part. Accordingly, the operator can easily understand which type of the amplification container should be set to which amplification container setting part, and thus, can set the amplification container of an appropriate type to each amplification container setting part.

In the nucleic acid analysis device (100) according to the present aspect, the controller (401) may be configured to cause the display unit (402) to display, when an amplification container (20) of a type corresponding to a sample to be processed is not set to an amplification container setting part (120), information indicating that the type of the amplification container (20) is not appropriate, as the relevant information.

By referring to the information indicating that the type of the amplification container is not appropriate, the operator can prevent the processing of the sample from being performed by use of a container of an inappropriate type. Therefore, by subsequently setting a container of an appropriate type, it is possible to perform an appropriate process on the sample. In addition, the operator can smoothly replace the wrong container having been set, with a container of an appropriate type.

In this case, the amplification container (20) may hold information that can specify the type of the container (20). The nucleic acid analysis device (100) according to the present aspect may include a reading unit (142) configured to read information regarding the type of the amplification container (20), from the amplification container (20) set to the amplification container setting part (120). The controller (401) may be configured to cause the display unit (402) to display, when the type of the amplification container (20) corresponding to a sample to be processed does not match the information read by the reading unit (142), information indicating that the type of the amplification container (20) set to the amplification container setting part (120) is not appropriate, as the relevant information.

The controller (401) may be configured to suspend a process on the sample to be processed when the amplification container (20) of the type corresponding to the sample is not set to the amplification container setting part. Accordingly, it is possible to more assuredly prevent the processing of the sample from being performed by use of a container of an inappropriate type. Therefore, by subsequently setting a container of an appropriate type, it becomes possible to perform an appropriate process on the sample. In addition, containers can be prevented from being wasted.

The nucleic acid analysis device (100) according to the present aspect may include a sensor (122) configured to detect whether or not the amplification container (20) is set to the amplification container setting part (120). The controller (401) may be configured to cause the display unit (402) to display, on the basis of a detection result by the sensor (122), information regarding whether or not the amplification container (20) is set to the amplification container setting part (120), as the relevant information.

Accordingly, by referring to the information regarding whether or not the amplification container is set, the information being displayed on the display unit, the operator can understand whether or not the amplification container is set to the amplification container setting part. Thus, when the amplification container is set, the operator can understand that it is not necessary to set the amplification container to the amplification container setting part, and when the amplification container is not set, the operator can understand that it is necessary to set the amplification container to the amplification container setting part. Therefore, the operator can appropriately and smoothly set the container.

In the nucleic acid analysis device (100) according to the present aspect, the controller (401) may be configured to cause the display unit (402) to display, as the relevant information, information that allows evaluation of whether or not the type of the container (20) set to the amplification container setting part (120) is appropriate for a sample to be processed. "Information that allows evaluation" denotes "sample ID", "information of container that should be set", "information of container actually set", or the like displayed on the screen. By referring to the relevant information displayed on the display unit, the operator can confirm whether or not the container set to the amplification container setting part is a container of an appropriate type. Thus, the operator can appropriately set a container of an appropriate type to each amplification container setting part.

In the nucleic acid analysis device (100) according to the present aspect, the controller (401) may be configured to cause the display unit (402) to display an operation portion (511, 521) for receiving a measurement item of a sample to be processed, when a predetermined operation has been performed on an image (520, 820) corresponding to a location of an amplification container setting part. For example, in a case where a display unit and an input unit are integrally formed as a touch panel, the predetermined operation is an operation of continuously touching, for a predetermined time, an image displayed on the touch panel. In a case where a display unit and an input unit are separately provided, the predetermined operation is a clicking operation or the like that is performed through the input unit onto an image displayed on the display unit. Accordingly, even when a measurement item has not been set in advance, a measurement item can be easily set later.

A second aspect of the present invention relates to a nucleic acid extraction device. A nucleic acid extraction device (900) according to the present aspect includes: a plurality of extraction container setting parts (110) each configured to have an extraction container (10) set thereto, the extraction container (10) storing a reagent for extracting nucleic acid from a sample, the extraction container (10) being configured to purify an extract containing the nucleic acid by use of the reagent; a display unit (402); and a controller (401) configured to cause the display unit (402) to display a screen in which a location of each of the plurality of extraction container setting parts (110) is associated with relevant information regarding setting of the extraction container (10) to the extraction container setting part (110).

In the nucleic acid extraction device according to the present aspect, the relevant information plays the role of a guide for the operator to set the extraction container to the extraction container setting part. Therefore, by referring to the relevant information displayed on the display unit, the operator can appropriately set an extraction container to each extraction container setting part. When the relevant information is displayed, even an operator who is not familiar with the nucleic acid extraction device can appropriately and smoothly set the extraction container.

In the nucleic acid extraction device (900) according to the present aspect, the controller (401) may be configured to cause the display unit (402) to display, as the relevant information, information regarding the type of the extraction container (10) that should be set to the extraction container setting part (110). The type of the extraction container denotes the type of the sample targeted by the reagent stored in the extraction container, the type of nucleic acid, or the like. The type of the sample is, for example, formalin-fixed paraffin-embedded tissue section, plasma, whole blood, or the like. The type of nucleic acid is, for example, DNA, RNA, or the like.

A third aspect of the present invention relates to a nucleic acid analysis device. A nucleic acid analysis device (100) according to the present aspect includes: an extraction container setting part (110) configured to have an extraction container (10) set thereto, the extraction container (10) storing a reagent for extracting nucleic acid from a sample, the extraction container (10) being configured to purify an extract containing the nucleic acid by use of the reagent; an amplification container setting part (120) configured to have an amplification container (20) set thereto, the amplification container (20) being configured to have injected therein the extract purified in the extraction container (10), the amplification container (20) storing a reagent for amplifying the nucleic acid in the extract; a display unit (402); and a controller (401) configured to cause the display unit (402) to display a screen in which a location of the extraction container setting part (110) is associated with first relevant information regarding setting of the extraction container (10) to the extraction container setting part (110), and a location of the amplification container setting part (120) is associated with second relevant information regarding setting of the amplification container (20) to the amplification container setting part (120).

In the nucleic acid analysis device according to the present aspect, the first relevant information and the second relevant information play the role of a guide for the operator to set the extraction container and the amplification container to the extraction container setting part and the amplification container setting part, respectively. Therefore, by referring to the first relevant information and the second relevant information displayed on the display unit, the operator can appropriately set the extraction container to the extraction container setting part, and can appropriately set the amplification container to the amplification container setting part. When the first relevant information and the second relevant information are displayed, even an operator who is not familiar with the nucleic acid analysis device can appropriately and smoothly set the extraction container and the amplification container.

### ADVANTAGEOUS EFFECTS OF THE INVENTION

According to the present invention, containers can be appropriately set to the device.

### BRIEF DESCRIPTION OF THE DRAWINGS

[FIG. 1] FIG. 1(a) is a schematic diagram showing a general configuration of the inside of a nucleic acid analysis device according to Embodiment 1, as viewed from above. FIG. 1(b) shows a screen displayed on a display unit according to Embodiment 1.
[FIG. 2] FIG. 2(a) shows an external view of a nucleic acid analysis device according to Embodiment 1. FIG. 2(b) shows an external view of the nucleic acid analysis device in a state where a cover is opened, according to Embodiment 1.
[FIG. 3] FIG. 3 is a schematic diagram showing a configuration of the inside of the nucleic acid analysis device according to Embodiment 1, as viewed from above.
[FIG. 4] FIG. 4 is a schematic diagram showing configurations of extraction container setting parts and amplification container setting parts provided to first to third lanes, according to Embodiment 1.
[FIG. 5] FIG. 5(a) is a schematic perspective view showing a configuration of an extraction container according to Embodiment 1. FIG. 5(b) is a schematic perspective view showing a configuration of an amplification container according to Embodiment 1.
[FIG. 6] FIGS. 6(a) and (b) are each a schematic side view showing a configuration of a nozzle according to Embodiment 1. FIG. 6(c) is a schematic diagram showing a configuration of a dispensing unit according to Embodiment 1.
[FIG. 7] FIG. 7(a) is a schematic cross-sectional view showing a configuration of a temperature adjustment part provided below the extraction container setting part according to Embodiment 1. FIGS. 7(b) and 7(c) are each a schematic diagram showing a configuration of a magnetic force application part according to Embodiment 1.
[FIG. 8] FIG. 8 is a schematic diagram showing a configuration of a detection unit according to Embodiment 1.
[FIG. 9] FIG. 9 is a block diagram showing a configuration of the nucleic acid analysis device according to Embodiment 1.
[FIG. 10] FIG. 10 is a flow chart showing a process up to the start of a measurement process out of processes performed by the nucleic acid analysis device according to Embodiment 1.
[FIG. 11] FIG. 11 is a flow chart showing a process up to the start of the measurement process out of processes performed by the nucleic acid analysis device according to Embodiment 1.
[FIG. 12] FIG. 12(a) shows a screen in a state before a sample ID is inputted, according to Embodiment 1. FIG. 12(b) shows a screen for inputting a sample ID, according to Embodiment 1.
[FIG. 13] FIG. 13(a) shows a screen in a state where one sample ID has been inputted, according to Embodiment 1. FIG. 13(b) is a schematic diagram showing a configuration of a measurement order table according to Embodiment 1.
[FIG. 14] FIG. 14(a) is a schematic diagram showing a configuration of a reagent table according to Embodiment 1. FIG. 14(b) is a schematic diagram showing a configuration of a modification of the reagent table according to Embodiment 1.
[FIG. 15] FIG. 15(a) shows a first operation portion and a second operation portion for inputting a measurement order according to Embodiment 1. FIG. 15(b) shows a screen at the time when an extraction container and an amplification container have been set in accordance with one inputted sample ID, according to Embodiment 1.
[FIG. 16] FIG. 16(a) shows a screen at the time when three sample IDs have been inputted and extraction containers and amplification containers have been set in accordance with the three sample IDs, according to Embodiment 1. FIG. 16(b) shows a progress screen displayed on the display unit according to Embodiment 1.
[FIG. 17] FIG. 17(a) shows a screen at the time when errors have occurred with respect to setting of containers, according to Embodiment 1. FIG. 17(b) shows a screen at the time when inappropriate containers have been removed after errors have occurred with respect to setting of containers, according to Embodiment 1.
[FIG. 18] FIG. 18 is a flow chart showing the measurement process out of processes performed by the nucleic acid analysis device according to Embodiment 1.
[FIG. 19] FIG. 19(a) shows a screen displayed on the display unit according to Modification 1 of Embodiment 1. FIG. 19(b) shows a screen displayed on the display unit according to Modification 2 of Embodiment 1.
[FIG. 20] FIG. 20 shows a screen that is long in a screen-vertical direction displayed on the display unit according to Modification 3 of Embodiment 1.
[FIG. 21] FIG. 21 is a flow chart showing a process up to the start of a measurement process out of processes performed by the nucleic acid analysis device according to Embodiment 2.
[FIG. 22] FIG. 22(a) shows a screen displayed on the display unit according to Embodiment 2. FIG. 22(b) shows a screen displayed on the display unit according to Modification 1 of Embodiment 2.
[FIG. 23] FIG. 23 shows a screen displayed on the display unit according to Modification 2 of Embodiment 2.
[FIG. 24] FIG. 24(a) is a schematic diagram showing a general configuration of the inside of a nucleic acid extraction device according to Embodiment 3, as viewed from above. FIG. 24(b) shows a screen displayed on the display unit according to Embodiment 3.
[FIG. 25] FIG. 25(a) is a schematic diagram showing a general configuration of the inside of the nucleic acid analysis device according to Embodiment 4, as viewed from above. FIG. 25(b) shows a screen displayed on the display unit according to Embodiment 4.
[FIG. 26] FIGS. 26(a), 26(b) are each a schematic diagram illustrating a configuration of related art.

### DESCRIPTION OF EMBODIMENTS

### <Embodiment 1>

Embodiment 1 is obtained by applying the present invention to a device that automatically performs processes of nucleic acid extraction, followed by real time PCR, detection of nucleic acid amplification reaction, and nucleic acid analysis.

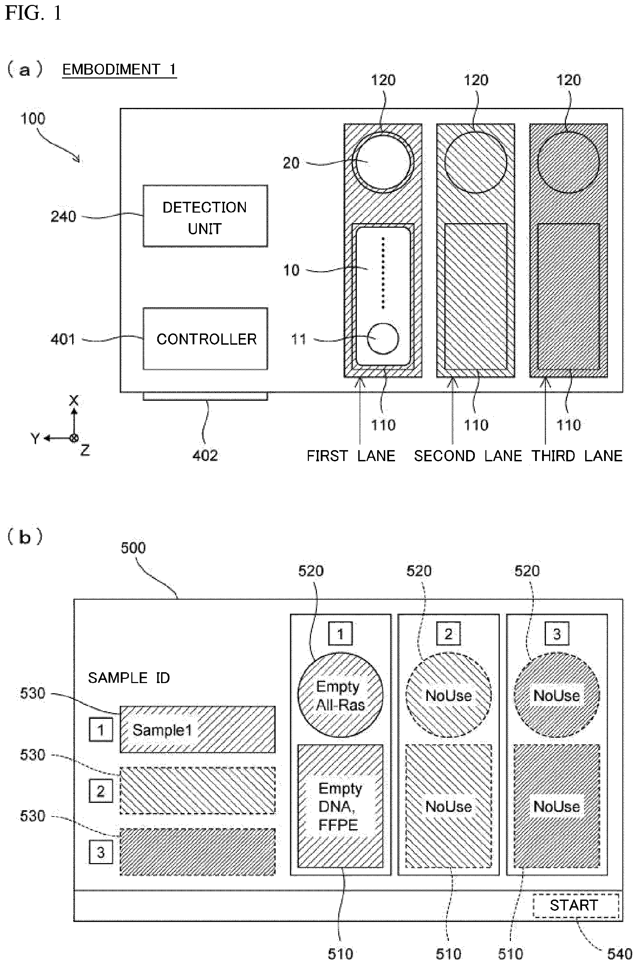

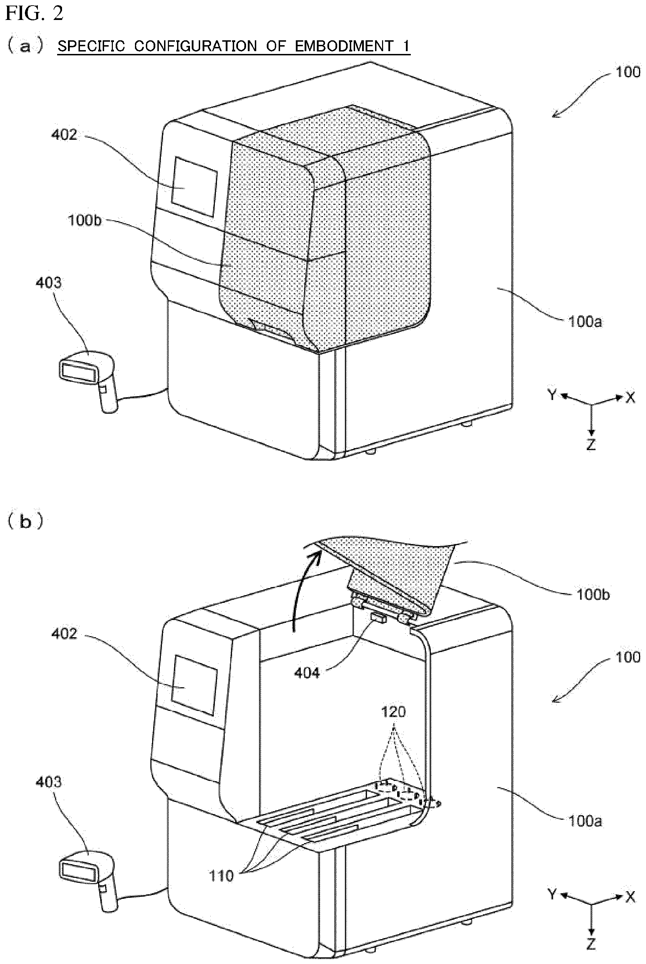

With reference to FIGS. 1(a), 1(b), an outline of a nucleic acid analysis device 100 is described. Specific configurations of the nucleic acid analysis device 100 will be described later with reference to the drawings of FIG. 2(a) and thereafter.

As shown in FIGS. 1(a), 1(b), the nucleic acid analysis device 100 includes three extraction container setting parts 110, three amplification container setting parts 120, a detection unit 240, a controller 401, and a display unit 402. In FIG. 1(a), XYZ axes are orthogonal to one another. The X-axis positive direction represents the rear direction, the Y-axis positive direction represents the left direction, and the Z-axis positive direction represents the vertically downward direction. Also in the subsequent drawings, the XYZ axes are the same as the XYZ axes shown in FIG. 1(a).

In the nucleic acid analysis device 100, three columns are provided in the Y-axis direction, and in each column, one extraction container setting part 110 and one amplification container setting part 120 are arranged along the X axis. The column at the left end, the column at the center, and the column at the right end will be referred to as "first lane", "second lane", and "third lane", respectively. The rectangular regions surrounding the extraction container setting part 110 and the amplification container setting part 120 in the lanes are respectively provided with different colors. Specifically, the first lane is colored with blue, the second lane is colored with green, and the third lane is colored with orange.

Each extraction container setting part 110 is a setting part for setting an extraction container 10. As described later, the extraction container 10 includes a plurality of reagent storage parts. The plurality of reagent storage parts of the extraction container 10 each store in advance a reagent for extracting nucleic acid from a sample. The extraction container 10 is a replaceable container to be used for extracting nucleic acid from a sample. In addition, as described later, the extraction container 10 includes a reaction part 11. A sample to be processed is stored in the reaction part 11. Nucleic acid is extracted from the sample stored in the reaction part 11, by using the reagents stored in the reagent storage parts of the extraction container 10. Hereinafter, a liquid that contains the nucleic acid extracted in the extraction container 10 will be referred to as "extract".

Each amplification container setting part 120 is a setting part for setting an amplification container 20. As described later, the amplification container 20 includes a plurality of storage parts. The plurality of storage parts of the amplification container 20 each store in advance a reagent for amplifying nucleic acid in the extract purified in the extraction container 10. The amplification container 20 is a replaceable container to be used for amplifying nucleic acid in the extract. The extract purified in the extraction container 10 is injected from an injection hole provided in the amplification container 20, into the amplification container 20. Then, the amplification container 20 having the extract injected therein is transferred to the detection unit 240. At the position of the detection unit 240, the nucleic acid contained in the extract is amplified by using the reagents stored in the storage parts of the amplification container 20.

The detection unit 240 detects a detection target nucleic acid amplified in the amplification container 20. The controller 401 controls the components of the nucleic acid analysis device 100, and performs analysis of nucleic acid by using measurement data obtained by the detection unit 240. The display unit 402 displays images and receives inputs from an operator.

FIG. 1(b) shows a screen 500 displayed on the display unit 402. The screen 500 includes three first images 510, three second images 520, three display regions 530, and a start button 540. When the operator starts processing of a sample, the operator causes the display unit 402 to display the screen 500. Hereinafter, with respect to the screen 500, the up-down direction will be referred to as "screen-vertical direction", and the left-right direction will be referred to as "screen-horizontal direction".

On the screen 500, three columns are provided in the screen-horizontal direction, and in each column, one first image 510 and one second image 520 are arranged along the screen-vertical direction. The column at the left end, the column at the center, and the column at the right end respectively correspond to the first lane, the second lane, and the third lane in the nucleic acid analysis device 100 shown in FIG. 1(a). The first image 510 and the second image 520 arranged in the screen-vertical direction are provided with the same colors as those of the corresponding lanes in the nucleic acid analysis device 100. Specifically, the first image 510 and the second image 520 corresponding to the first lane are colored with blue, the first image 510 and the second image 520 corresponding to the second lane are colored with green, and the first image 510 and the second image 520 corresponding to the third lane are colored with orange.

The one first image 510 and the one second image 520 arranged in the screen-vertical direction are in a rectangular region so as to indicate that the first image 510 and the second image 520 are related to each other. An upper portion of this region is provided with a number that indicates which of the first to third lanes this region corresponds to. Thus, in terms of arrangement, the first image 510 corresponds to the extraction container setting part 110, and the second image 520 corresponds to the amplification container setting part 120.

Each display region 530 is a region for displaying identification information of a sample. In Embodiment 1, identification information of a sample is information that can individually identify a sample, and is a sample ID in the form of a character string of digits, alphabets, and the like. Each display region 530 has a rectangular shape that is long in the screen-horizontal direction. The three display regions 530 are disposed so as to be arranged in the screen-vertical direction, at a position in the screen-horizontal direction with respect to the first images 510 and the second images 520. In each display region 530, the sample ID is indicated in the screen-horizontal direction. When a sample ID has been inputted by the operator, the inputted sample ID is displayed in a display region 530. The three display regions 530 are respectively provided, from the top in order, with the same colors as those of the first images 510 and the second images 520 corresponding to the first to third lanes. Specifically, the display region 530 on the top is colored with blue, the display region 530 at the center is colored with green, and the display region 530 on the bottom is colored with orange.

The controller 401 associates a first image 510 with first relevant information regarding setting of an extraction container 10 to the corresponding extraction container setting part 110, and causes the screen 500 to display the first image 510 and the first relevant information. Specifically, the controller 401 causes the display unit 402 to display, as the first relevant information: information indicating whether or not an extraction container 10 is set to the extraction container setting part 110; the type of the extraction container 10 that should be set to the extraction container setting part 110; information indicating that, when an extraction container 10 of a wrong type is set to the extraction container setting part 110, the type of the extraction container 10 is wrong; and the like. In addition, as the first relevant information, the controller 401 provides the first image 510 with the same color as that of the corresponding display region 530, and causes the display unit 402 to display the first image 510.

Similarly, the controller 401 associates a second image 520 with second relevant information regarding setting of an amplification container 20 to the corresponding amplification container setting part 120, and causes the screen 500 to display the second image 520 and the second relevant information. Specifically, the controller 401 causes the display unit 402 to display, as the second relevant information: information indicating whether or not an amplification container 20 is set to the amplification container setting part 120; the type of the amplification container 20 that should be set to the amplification container setting part 120; information indicating that, when an amplification container 20 of a wrong type is set to the amplification container setting part 120, the type of the amplification container 20 is wrong; and the like. In addition, as the second relevant information, the controller 401 provides the second image 520 with the same color as that of the corresponding display region 530, and causes the display unit 402 to display the second image 520.

In the example shown in FIG. 1(b), a sample ID is displayed in the display region 530 that corresponds to the first lane. A character string "DNA, FFPE" is displayed as the first relevant information in the first image 510 corresponding to the first lane. A character string "All-Ras" is displayed as the second relevant information in the second image 520 that corresponds to the first lane. "DNA, FFPE" indicates the type of the extraction container 10 that should be set in the extraction container setting part 110. "All-Ras" indicates the type of the amplification container 20 that should be set in the amplification container setting part 120.

When the type of the extraction container 10 that should be set is displayed as shown in FIG. 1(b), the operator can understand that an extraction container 10 storing a reagent for extracting DNA from a formalin-fixed paraffin-embedded tissue section should be set to the extraction container setting part 110 of the first lane. When the type of the amplification container 20 that should be set is displayed as shown in FIG. 1(b), the operator can understand that an amplification container 20 storing a reagent to be used for a measurement item "All-Ras" should be set to the amplification container setting part 120 of the first lane.

In the example shown in FIG. 1(b), "Empty" is displayed in the first image 510 and the second image 520 corresponding to the first lane. "Empty" indicates a state where no container is set to the corresponding container setting part. When "Empty" is displayed, the operator can understand that no container is set to the corresponding container setting part. In the example shown in FIG. 1(b), no sample ID is displayed in the display regions 530 corresponding to the second and third lanes. Thus, "No Use" is displayed in the first images 510 and the second images 520 corresponding to the second and third lanes. When "No Use" is displayed, the operator can understand that the corresponding container setting part is not used.

The operator stores a sample into the reaction part 11 of an appropriate extraction container 10, sets this extraction container 10 to the extraction container setting part 110, and sets an appropriate amplification container 20 to the amplification container setting part 120. Then, the operator operates the start button 540 to start processing the sample. After the processing on the sample is started, an extract is purified in the extraction container 10, and the purified extract is injected into the amplification container 20. Then, the detection target nucleic acid amplified in the amplification container 20 is detected in the detection unit 240.

As described above, the first relevant information regarding setting of the extraction container 10 to the extraction container setting part 110, and the second relevant information regarding setting of the amplification container 20 to the amplification container setting part 120 play the role of a guide for the operator to set the extraction container 10 and the amplification container 20 to the extraction container setting part 110 and the amplification container setting part 120, respectively. Therefore, by referring to the first relevant information and the second relevant information, the operator can appropriately set the extraction container 10 to the extraction container setting part 110, and can appropriately set the amplification container 20 to the amplification container setting part 120. When the first relevant information and the second relevant information are displayed, even an operator who is not familiar with the nucleic acid analysis device 100 can appropriately and smoothly set the extraction container 10 and the amplification container 20.

In each display region 530, the sample ID is indicated in the screen-horizontal direction, and the display regions 530 corresponding to the respective lanes are disposed so as to be arranged in the screen-vertical direction, at a position in the screen-horizontal direction with respect to the first image 510 and the second image 520. Accordingly, the first images 510, the second images 520, and the display regions 530 can be efficiently displayed within the screen 500 having limited dimensions. In Embodiment 1, the first image 510, the second image 520 and the display region 530 are displayed without being arranged in a line. However, as shown in FIG. 1(b), since the first image 510 and the second image 520 of each lane and the display region 530 corresponding to the lane are linked with each other by means of a color, a lane number, and the like, the operator can easily understand the correspondence relationship between the first image 510 and the second image 520, and the sample ID.

In Embodiment 1, the extraction container setting part 110, the amplification container setting part 120, the first image 510, the second image 520, and the display region 530 are colored with blue, green, or orange in accordance with the lane. However, as long as the operator can understand the correspondence relationship, the kinds of colors are not limited thereto. Not limited to being colored, for example, each portion colored as above may be provided with a marking or the like that allows identification of the lane. Alternatively, each portion colored as above may have a characteristic shape for each lane. When character strings are displayed as the first relevant information and the second relevant information, the character strings may not necessarily be displayed in the first image 510 and the second image 520 as shown in FIG. 1(b). For example, the character string of the first relevant information may be displayed in the vicinity of the first image 510, and the character string of the second relevant information may be displayed in the vicinity of the second image 520.

### <Specific configuration of Embodiment 1>

In the following, a specific configuration of Embodiment 1 is described.

As shown in FIGS. 2(a), 2(b), the nucleic acid analysis device 100 includes a housing 100a, a cover 100b, the display unit 402, a reading unit 403, and a limit-type sensor 404. The housing 100a covers the inside of the nucleic acid analysis device 100. The cover 100b is provided to the housing 100a in order to allow the inside of the nucleic acid analysis device 100 to be open to the outside. The inside of the nucleic acid analysis device 100 may be open to the outside in advance, with the cover 100b omitted.

The display unit 402 is implemented as a touch panel-type display. Instead of the display unit 402, the nucleic acid analysis device 100 may include a display unit for displaying an image and an input unit for receiving an input from the operator, separately. The reading unit 403 reads a sample ID from a bar code label attached to a sample container (not shown). The reading unit 403 is implemented as a bar code reader. When the sample container has an RFID tag, the reading unit 403 is implemented as an antenna for reading an RFID.

As shown in FIG. 2(b), when the cover 100b is rotated upward, the inside of the nucleic acid analysis device 100 is open to the outside. The extraction container setting parts 110 and the amplification container setting parts 120 shown in FIG. 1(a) are provided inside the nucleic acid analysis device 100. By opening the cover 100b, the operator can set and take out containers with respect to the extraction container setting parts 110 and the amplification container setting parts 120. When the cover 100b is closed, the sensor 404 is pushed in by the cover 100b. When the cover 100b is opened, pushing-in of the sensor 404 by the cover 100b is canceled. Accordingly, the controller 401 detects an open/closed state of the cover 100b.

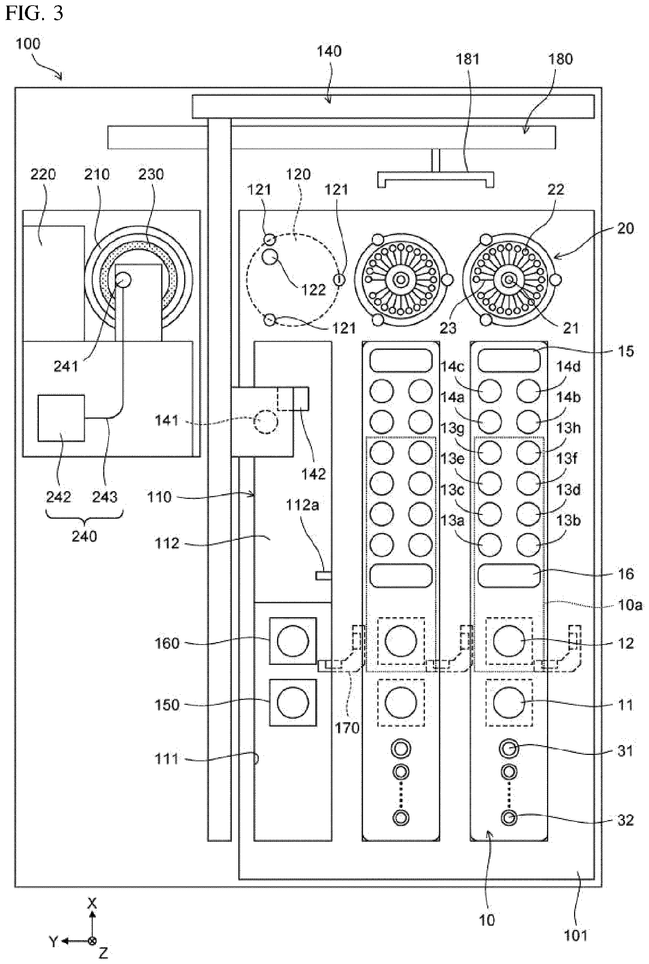

Next, with reference to FIG. 3 to FIG. 8, the configuration of the inside of the nucleic acid analysis device 100 is described in detail.

As shown in FIG. 3, the nucleic acid analysis device 100 includes a plate member 101, a dispensing unit 140, temperature adjustment parts 150, 160, a magnetic force application part 170, a transfer unit 180, a container setting part 210, a rotation drive unit 220, a temperature adjustment part 230, and the detection unit 240. The plate member 101 is parallel to the XY plane. The plate member 101 is provided with three extraction container setting parts 110 and three amplification container setting parts 120.

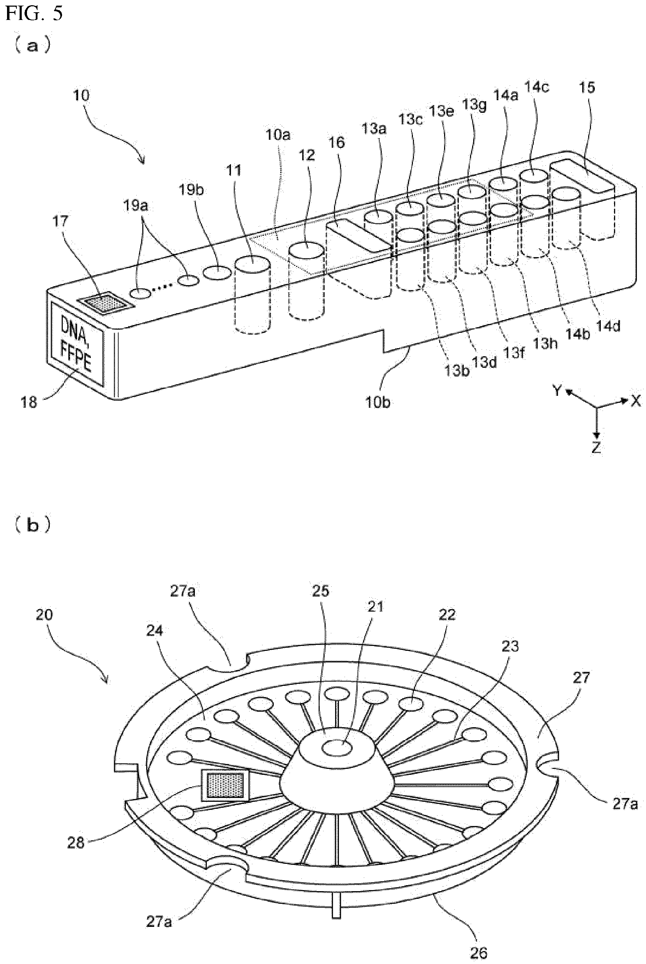

Each extraction container setting part 110 is a recess formed by: an opening 111 formed in the plate member 101; a support plate 112 on the vertically lower side of the plate member 101; and plate members (not shown) that are provided at the left, right, and rear of the support plate 112 and that are perpendicular to the Z-axis direction. In a plan view, the opening 111 and the support plate 112 each have a contour slightly larger than the outer shape of the extraction container 10. The support plate 112 is provided on the rear side of the opening 111. A lower end portion 10b of the extraction container 10 shown in FIG. 5(a) is supported in the vertically upward direction by the support plate 112, and the side faces of the extraction container 10 are supported by the opening 111 and the plate members provided at the left, right, and rear of the support plate 112, whereby the extraction container 10 is set to the extraction container setting part 110.

The support plate 112 is provided with a first sensor 112a. The first sensor 112a is implemented as a limit-type sensor. When an extraction container 10 is set to the extraction container setting part 110, the first sensor 112a is pushed in the vertically downward direction by the lower end portion 10b of the extraction container 10. Accordingly, the controller 401 detects whether or not the extraction container 10 is set to the extraction container setting part 110.

Each amplification container setting part 120 is formed by: the upper face of the plate member 101 and three pins 121 provided on the upper face of the plate member 101. To-be-engaged portions 27a shown in FIG. 5(b) are engaged with the three pins 121, whereby an amplification container 20 is set to the amplification container setting part 120. An opening is provided in the upper face of the plate member 101 where the amplification container 20 is set. At the position of this opening, a second sensor 122 is provided on the vertically lower side relative to the upper face of the plate member 101. The second sensor 122 is implemented as a reflection-type sensor. When the amplification container 20 is set to the amplification container setting part 120, light emitted from the second sensor 122 is reflected by the lower face of the amplification container 20. Accordingly, the controller 401 detects whether or not the amplification container 20 is set to the amplification container setting part 120.

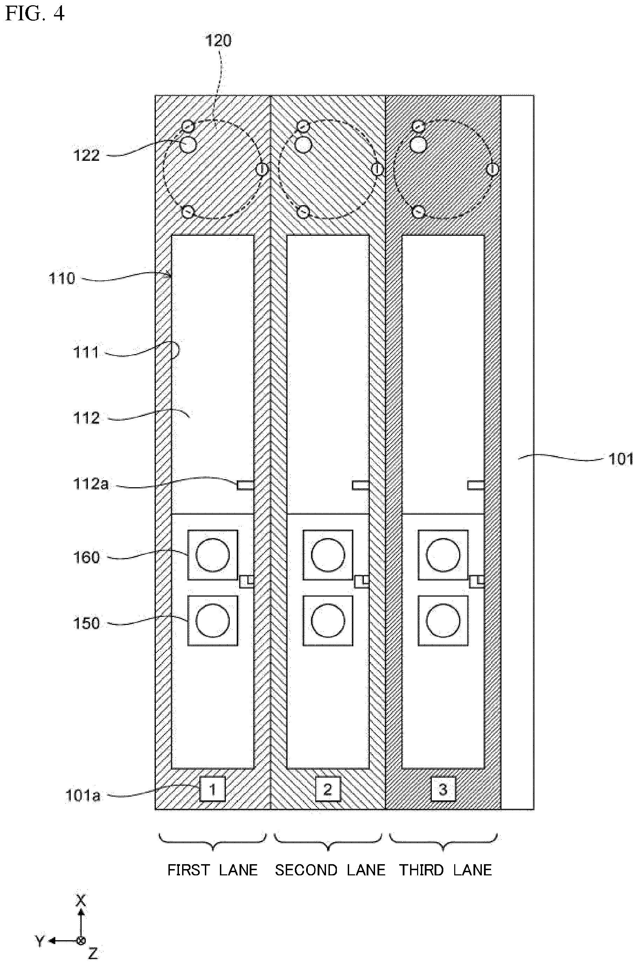

As shown in FIG. 4, three columns are provided in the Y-axis direction, and in each column, one extraction container setting part 110 and one amplification container setting part 120 are arranged along the X axis. Also in FIG. 4, as in FIG. 1(a), the column at the left end, the column at the center, and the column at the right end are the first to third lanes, respectively. In the lanes, the rectangular regions surrounding the extraction container setting part 110 and the amplification container setting part 120 are respectively provided with different colors, as in FIG. 1(a). Also in FIG. 4, as in FIG. 1(a), the first to third lanes are colored with blue, green, and orange, respectively. A label 101a indicating a lane number is provided on the upper face of the plate member 101 of each of the first to third lanes, so as to allow the operator to visually recognize the lane number. The configurations of the components of the first to third lanes are the same with one another, except for the colors of the lanes and the digits provided to the labels 101a.

As shown in FIG. 3 and FIG. 5(a), the extraction container 10 includes the reaction part 11, a reagent storage part 12, reagent storage parts 13a to 13h, mixing parts 14a to 14d, a reagent storage part 15, a waste liquid storage part 16, a bar code label 17, a label 18, and holes 19a, 19b. The reaction part 11, the reagent storage part 12, the reagent storage parts 13a to 13h, the mixing parts 14a to 14d, the reagent storage part 15, and the waste liquid storage part 16 are provided in the extraction container 10 such that the upper portions thereof are open, and are each a well that can store a liquid. A two-dimensional bar code including identification information of the extraction container 10 is printed on the bar code label 17. The identification information of the extraction container 10 includes character information that can specify the type of the extraction container 10, for example, character information "DNA, FFPE". A character string that can specify the type of the extraction container 10, for example, "DNA, FFPE", is indicated on the label 18 attached to the outer side face of the extraction container 10. Each hole 19a is provided so as to hold a pipette tip 32, and the hole 19b is provided so as to hold a puncture tip 31.

Instead of the bar code label 17, the extraction container 10 may be provided with an RFID tag having identification information of the extraction container 10 stored therein. The type of the extraction container 10 may be specified by a special structure such as a cut-out or a hole formed in the extraction container 10.

The reagent storage parts 12, 13a to 13h each store a reagent for nucleic acid extraction in advance. The reagents stored in the extraction container 10 are reagents corresponding to the type of the extraction container 10. The type of the extraction container 10 is determined on the basis of the type of nucleic acid to be extracted in the extraction container 10, and the type of the sample set in the extraction container 10. The type of nucleic acid to be extracted is DNA, RNA, or the like. The type of the sample is, for example, formalin-fixed paraffin-embedded tissue section, plasma, whole blood, or the like. The types of the sample are not limited thereto. Hereinafter, the formalin-fixed paraffin-embedded tissue section will be referred to as "FFPE section".

For example, when the type of the extraction container 10 is "DNA, FFPE", the extraction container 10 is used in order to extract DNA from an FFPE section. In this case, the reagent storage part 12 stores in advance a reagent that contains magnetic particles and a magnetic particle preservative liquid. The reagent storage parts 13a to 13h store in advance a solubilizing liquid, proteinase K, an oil, an elution liquid, a stock solution of a reagent for extraction, a stock solution of a second washing liquid, a stock solution of a diluent, and a stock solution of a first washing liquid. When the type of the extraction container 10 is "DNA, plasma", the extraction container 10 is used in order to extract DNA from plasma. In this case, the respective reagent storage parts store the same reagents as those in the case where the type is "DNA, FFPE". When the type of the extraction container 10 is "RNA, whole blood", the extraction container 10 is used in order to extract RNA from whole blood. In this case, proteinase K is not necessary, and thus, the reagent storage part 13b does not store any reagent, and the other reagent storage parts store the same reagent as those in the case where the type is "DNA, FFPE".

In Embodiment 1, the same reagents are stored in the case of the type of "DNA, FFPE" and in the case of the type of "DNA, plasma". However, the reagents may be different between these cases. The type of the extraction container 10 is determined on the basis of the type of nucleic acid and the type of the sample, but may be determined on the basis of the type of nucleic acid, the type of the sample, and the measurement item.

The upper portions of the reagent storage part 12, the reagent storage parts 13a to 13h, and the waste liquid storage part 16 are closed by an aluminium seal 10a. When setting the extraction container 10 to the extraction container setting part 110, the operator stores a sample into the reaction part 11, and stores ethanol into the reagent storage part 15.

As shown in FIG. 3 and FIG. 5(b), the amplification container 20 includes: an injection hole 21; twenty-three storage parts 22; and twenty-three flow paths 23 connecting the injection hole 21 and the twenty-three storage parts 22 to each other. The amplification container 20 is a disk-like container in which: the injection hole 21 is disposed at the center position; and the twenty-three storage parts 22 are disposed at constant intervals in the circumferential direction, at positions on the outer periphery side at a constant distance from the center position. The amplification container 20 may not necessarily be a disk-like container.

More specifically, the amplification container 20 includes an upper face portion 24, a protrusion 25, a lower face portion 26, a flange portion 27, and a bar code label 28. The protrusion 25 is disposed at the center position of the amplification container 20. The injection hole 21 is a hole provided in the protrusion 25 and being parallel to the vertical direction. The upper face portion 24 is formed by a translucent member. The upper face of the upper face portion 24 is a plane parallel to the horizontal plane. Recesses and grooves for respectively forming the storage parts 22 and the flow paths 23 are formed in the lower face of the upper face portion 24. A thin-film-shaped ABS resin is attached to the lower face of the upper face portion 24, whereby the storage parts 22 and the flow paths 23 are formed. The lower face portion 26 is formed by a thin-film-shaped aluminium having high thermal conductivity. The lower face portion 26 is attached from the lower side to the ABS resin attached to the lower face of the upper face portion 24.

The flange portion 27 is a flat plate formed on the outer side of the upper face portion 24 and being parallel to the horizontal plane. The to-be-engaged portions 27a are formed at three positions in the flange portion 27. Each to-be-engaged portion 27a is formed as a cut-out. The to-be-engaged portions 27a are engaged with the pins 121 of the amplification container setting part 120. An extract purified in the extraction container 10 of the same lane is injected into the injection hole 21.

Each storage part 22 stores in advance a reagent for amplifying nucleic acid in the extract. Specifically, each storage part 22 stores in advance a reagent that causes amplification of a detection target nucleic acid in which a mutation has occurred at a detection target site of the nucleic acid, and a reagent containing a fluorescent probe that binds to the detection target nucleic acid. The fluorescent probe includes a fluorescent substance. The detection target sites for the respective storage parts 22 are different from one another. The reagents stored in the amplification container 20 are reagents corresponding to the type of the amplification container 20. The type of the amplification container 20 is determined on the basis of the measurement item of the measurement to be performed by using the amplification container 20. The measurement item is, for example, All-Ras, EGFR, ESR1, BRAF, leukemia, or the like. The measurement items are not limited thereto.

For example, when the type of the amplification container 20 is "All-Ras", the amplification container 20 is used in order to perform measurement with respect to colon cancer. In this case, each storage part 22 stores a reagent for amplifying and detecting a detection target nucleic acid related to colon cancer. When the type of the amplification container 20 is "EGFR", the amplification container 20 is used in order to perform measurement with respect to lung cancer. In this case, each storage part 22 stores a reagent for amplifying and detecting a detection target nucleic acid related to lung cancer. When the type of the amplification container 20 is "ESR1", the amplification container 20 is used in order to perform measurement with respect to breast cancer. In this case, each storage part 22 stores a reagent for amplifying and detecting a detection target nucleic acid related to breast cancer. When the type of the amplification container 20 is "BRAF", the amplification container 20 is used in order to perform measurement with respect to skin cancer. In this case, each storage part 22 stores a reagent for amplifying and detecting a detection target nucleic acid related to skin cancer. When the type of the amplification container 20 is "leukemia", the amplification container 20 is used in order to perform measurement with respect to leukemia. In this case, each storage part 22 stores a reagent for amplifying and detecting a detection target nucleic acid related to leukemia.

In Embodiment 1, the type of the amplification container 20 is determined on the basis of the measurement item. However, the type of the amplification container 20 may be determined on the basis of the type of nucleic acid, the type of the sample, and the measurement item.

Due to the centrifugal force caused by rotation of the amplification container 20, the extract injected through the injection hole 21 is substantially evenly transferred to the twenty-three storage parts 22. Accordingly, analyses regarding nucleic acid can be performed in parallel in the twenty-three storage parts 22. Thus, efficiency of analysis can be improved.

A two-dimensional bar code that includes identification information of the amplification container 20 is printed on the bar code label 28. The identification information of the amplification container 20 includes character information that can specify the type of the amplification container 20, for example, character information "EGFR". Instead of the bar code label 28, the amplification container 20 may be provided with an RFID tag having the identification information of the amplification container 20 stored therein. The type of the amplification container 20 may be specified by a special structure such as a cut-out or a hole formed in the amplification container 20. A character string, for example, "EGFR", that can specify the type of the amplification container 20 may be indicated on the package of the amplification container 20.

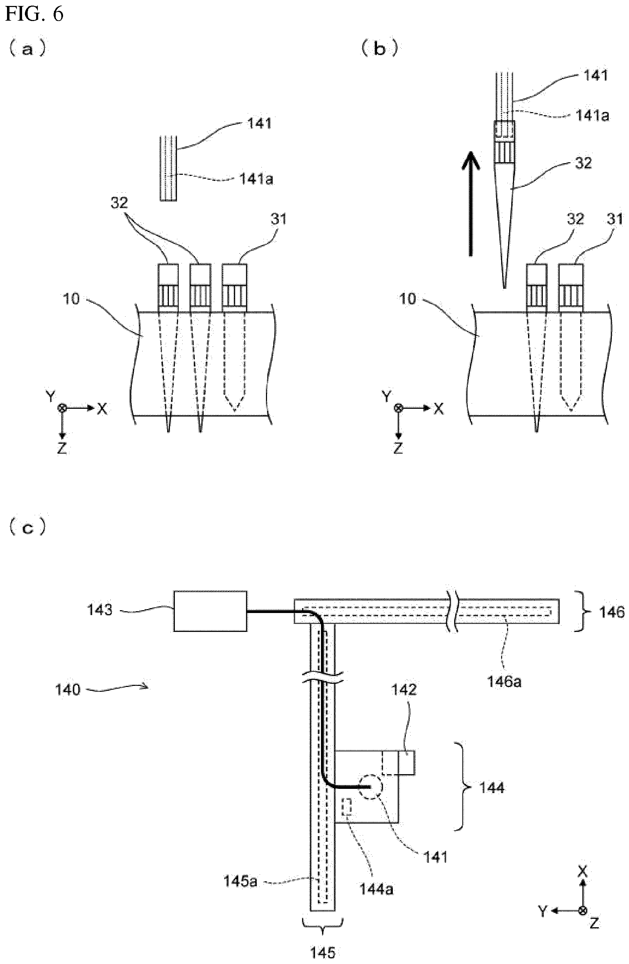

As shown in FIG. 3 and FIG. 6(a), the extraction container 10 holds one puncture tip 31 and seven pipette tips 32. The puncture tip 31 is a tip for piercing the aluminium seal 10a of the extraction container 10 to open the upper portion of each storage part which is on the lower side of the aluminium seal 10a. Each pipette tip 32 has a hole penetrating the pipette tip 32 in the vertical direction. As shown in FIGS. 6(a), (b), when a nozzle 141 of the dispensing unit 140 is lowered from directly above the pipette tip 32, the pipette tip 32 is attached to the lower end of the nozzle 141. Then, the nozzle 141 is raised, whereby the pipette tip 32 is pulled out from the extraction container 10. The puncture tip 31 is attached to the lower end of the nozzle 141 in a similar manner. A hole 141a is formed in the nozzle 141 so as to allow a liquid to be suctioned and discharged from the lower end of the nozzle 141.

As shown in FIG. 3 and FIG. 6(c), the dispensing unit 140 includes the nozzle 141, a reading unit 142, a pump 143, an up-down transfer part 144, a front-rear transfer part 145, and a left-right transfer part 146.

The nozzle 141 can have the puncture tip 31 and the pipette tip 32 attached/detached thereto. The reading unit 142 reads identification information from the bar code label 17 of the extraction container 10 set to the extraction container setting part 110, and reads identification information from the bar code label 28 of the amplification container 20 set to the amplification container setting part 120. The reading unit 142 is implemented as a bar code reader. When the extraction container 10 and the amplification container 20 are each provided with an RFID tag, the reading unit 142 is implemented as an antenna for reading an RFID. When the type information is held in the form of a special structure formed in the extraction container 10 and the amplification container 20, the reading unit 142 is implemented as a switch, a camera, or the like for discerning the shape of the special structure.

The pump 143 is connected to the hole 141a of the nozzle 141. The pump 143 applies a positive pressure or a negative pressure to the nozzle 141, thereby causing a liquid to be suctioned and discharged through the pipette tip 32 attached to the lower end of the nozzle 141.

The up-down transfer part 144 includes a rail 144a extending along the Z axis and a stepping motor (not shown). The up-down transfer part 144 drives the stepping motor to transfer the nozzle 141 in the Z-axis direction along the rail 144a. The reading unit 142 is fixed to the up-down transfer part 144. The front-rear transfer part 145 includes a rail 145a extending along the X axis, and a stepping motor (not shown). The front-rear transfer part 145 drives the stepping motor to transfer the up-down transfer part 144 in the X axis direction along the rail 145a. The left-right transfer part 146 includes a rail 146a extending along the Y axis, and a stepping motor (not shown). The left-right transfer part 146 drives the stepping motor to transfer the front-rear transfer part 145 in the Y-axis direction along the rail 146a.

Thus, the nozzle 141 can move along the XYZ axes inside the nucleic acid analysis device 100, due to the up-down transfer part 144, the front-rear transfer part 145, and the left-right transfer part 146. The reading unit 142 can move along the XY axes inside the nucleic acid analysis device 100 due to the front-rear transfer part 145 and the left-right transfer part 146.

The dispensing unit 140 performs a dispensing operation through the pipette tip 32 attached to the nozzle 141, thereby transferring the sample and each reagent in the extraction container 10. The dispensing unit 140 performs a dispensing operation through a pipette tip 32 attached to the nozzle 141, thereby transferring the extract purified in the extraction container 10, to the amplification container 20.

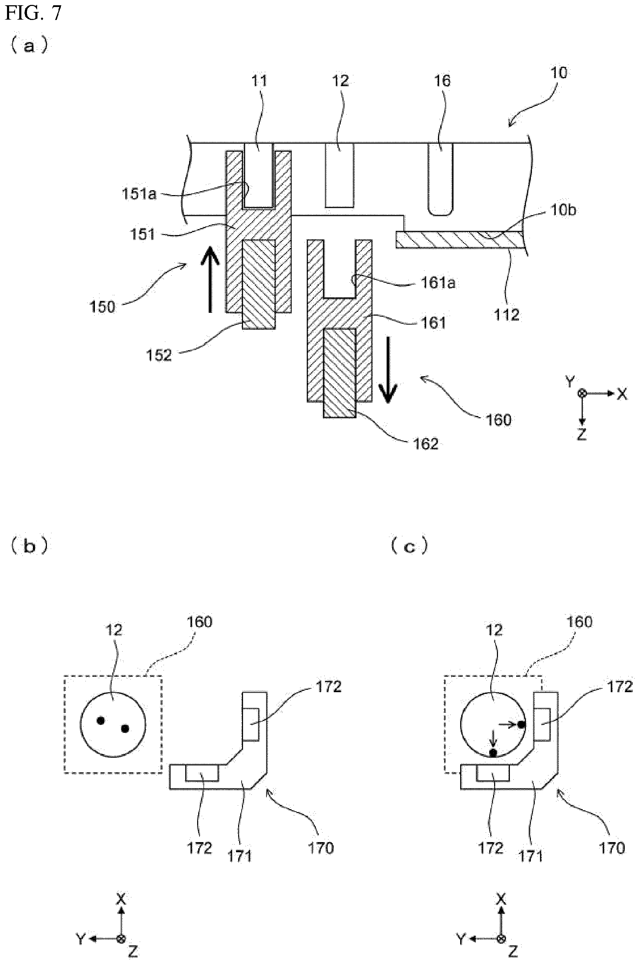

As shown in FIG. 3, in a plan view, the temperature adjustment parts 150, 160 are disposed at frontward positions in the opening 111 of the extraction container setting part 110. As shown in FIG. 7(a), the temperature adjustment part 150 includes a heat block 151 and a heater 152, and heats the reaction part 11 of the extraction container 10 set to the extraction container setting part 110. A hole 151a having substantially the same shape as the shape of the reaction part 11 is formed in the heat block 151. When the reaction part 11 is to be heated, the temperature adjustment part 150 is moved upward, and the reaction part 11 is accommodated in the hole 151a. In this state, heat of the heater 152 is transferred to the reaction part 11 via the heat block 151. After heating of the reaction part 11 ends, the temperature adjustment part 150 is moved downward.

Similarly, the temperature adjustment part 160 includes a heat block 161 and a heater 162, and heats the reagent storage part 12 of the extraction container 10 set to the extraction container setting part 110. When the reagent storage part 12 is to be heated, the temperature adjustment part 160 is moved upward, and the reagent storage part 12 is accommodated in a hole 161a. In this state, heat of the heater 162 is transferred to the reagent storage part 12 via the heat block 161. After heating of the reagent storage part 12 ends, the temperature adjustment part 160 is moved downward.

As shown in FIG. 3, the magnetic force application part 170 is disposed on the vertically lower side of the plate member 101, and is configured to be able to move in the Y-axis direction. As shown in FIGS. 7(b), 7(c), the magnetic force application part 170 includes a support portion 171 and two magnets 172. When the magnetic force application part 170 is used, the temperature adjustment part 160 is withdrawn in the vertically downward direction, as shown in FIG. 7(a). Then, as shown in FIG. 7(c), the magnetic force application part 170 is brought close to the reagent storage part 12 of the extraction container 10 set to the extraction container setting part 110. Accordingly, magnetic particles contained in the reagent storage part 12 as shown in FIG. 7(b) are attracted to the magnets 172 as shown in FIG. 7(c), to attach to the wall surface on the X-axis negative side and the wall surface on the Y-axis negative side of the reagent storage part 12.

As shown in FIG. 3, the transfer unit 180 includes a hand portion 181 and a mechanism for moving the hand portion 181 along the Y-axis direction. The transfer unit 180 grips and transfers the amplification container 20 between the amplification container setting part 120 and the container setting part 210. Instead of gripping and transferring the amplification container 20 by means of the hand portion 181, the transfer unit 180 may suction the upper face of the upper face portion 24 of the amplification container 20 by means of a suction portion, and transfer the amplification container 20.

The container setting part 210 has a cylindrical shape. The inside of the container setting part 210 is configured such that the amplification container 20 can be set therein. In order to send an extract by centrifugal force to the storage parts 22, the rotation drive unit 220 causes the amplification container 20 set to the container setting part 210, to rotate. Specifically, the rotation drive unit 220 applies driving force to the outer side face of the container setting part 210, thereby causing the container setting part 210 to rotate. Accordingly, the amplification container 20 set to the container setting part 210 is rotated, and the extract injected through the injection hole 21 is sent via the flow paths 23 to the storage parts 22 by the centrifugal force. As a result, in the storage parts 22 of the amplification container 20 set to the container setting part 210, nucleic acid contained in the extract is mixed with the reagents stored in advance in the storage parts 22.

As described above, each storage part 22 stores in advance a reagent that causes amplification of a detection target nucleic acid in which a mutation has occurred at a detection target site of the nucleic acid, and a reagent containing a fluorescent probe that binds to the detection target nucleic acid. When the fluorescent probe binds to the detection target nucleic acid, the detection target nucleic acid is labeled by the fluorescent substance. In a case where the fluorescent probe has bound to the detection target nucleic acid, when excitation light is applied to the fluorescent substance of the fluorescent probe, fluorescence is generated from the fluorescent substance. On the other hand, in a case where the fluorescent probe has not bound to the detection target nucleic acid, even when excitation light is applied to the fluorescent substance of the fluorescent probe, fluorescence is not generated from the fluorescent substance.

The temperature adjustment part 230 adjusts the temperature of the amplification container 20 set to the container setting part 210, such that nucleic acid amplification reaction is caused in each storage part 22. When the detection target nucleic acid is included in nucleic acid, the detection target nucleic acid is amplified in the storage part 22. When the detection target nucleic acid is not included in nucleic acid, the detection target nucleic acid is not amplified in the storage part 22. Therefore, when the detection target nucleic acid has been amplified, the amplified detection target nucleic acid is labeled by the fluorescent substance of the fluorescent probe. Thus, when excitation light is applied to the storage part 22, fluorescence is generated in accordance with the amplified amount.

The rotation drive unit 220 causes the container setting part 210 to rotate, thereby rotating the amplification container 20, to sequentially position each storage part 22 of which the temperature has been adjusted, to the position of the detection performed by the detection unit 240.

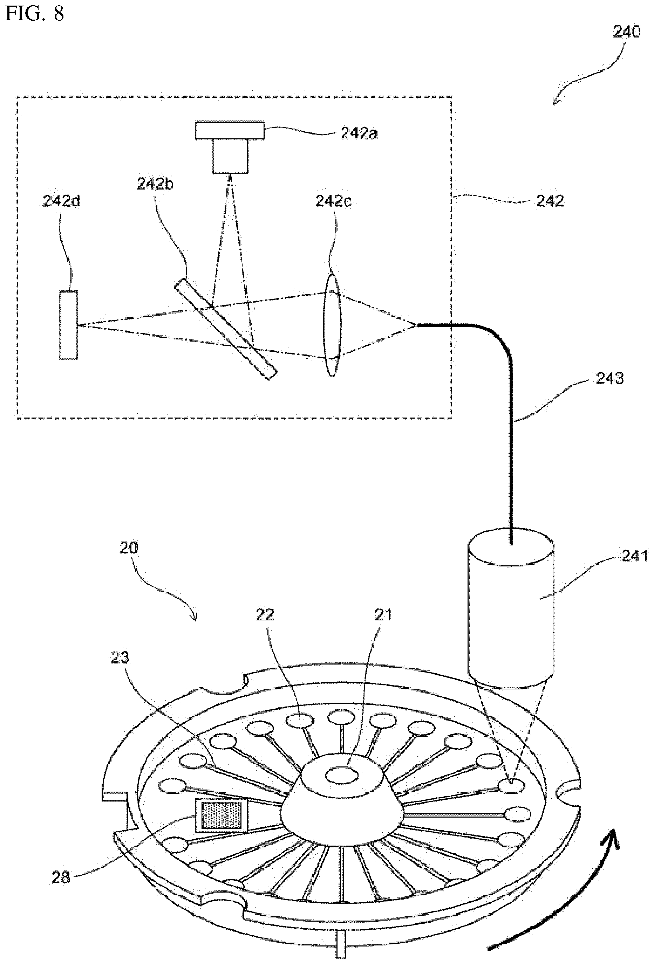

As shown in FIG. 3 and FIG. 8, the detection unit 240 includes a detection head 241, an optical unit 242, and an optical fiber 243. The detection unit 240 applies light to the storage part 22 of the amplification container 20 positioned at the detection position, and detects nucleic acid amplification reaction occurring in the storage part 22. Specifically, the detection unit 240 detects the intensity of a fluorescence signal indicating the amount of an amplification product by the nucleic acid amplification reaction. The detection head 241 is disposed so as to apply light to the storage part 22 and be opposed to the storage part 22 of the amplification container 20. The optical unit 242 includes a light source 242a, a dichroic mirror 242b, a condenser lens 242c, and a light detector 242d.

The light source 242a emits excitation light having a predetermined wavelength. In a case where the fluorescent probe has bound to a substance to be detected, the excitation light emitted from the light source 242a excites the fluorescent substance of the fluorescent probe, thereby causing fluorescence to be generated. The dichroic mirror 242b reflects the excitation light emitted from the light source 242a and allows fluorescence generated from the fluorescent substance of the fluorescent probe to be transmitted therethrough. The condenser lens 242c collects the excitation light reflected by the dichroic mirror 242b, to be guided to the optical fiber 243. In addition, the condenser lens 242c collects the fluorescence emitted from the optical fiber 243 to the condenser lens 242c, to be guided to the dichroic mirror 242b. The light detector 242d receives the fluorescence having been transmitted through the dichroic mirror 242b, and measures the intensity of the received fluorescence, and outputs an electric signal according to the intensity of the fluorescence.

On the basis of electric signals of the fluorescence detected by the light detector 242d of the detection unit 240, the controller 401 generates a plurality of pieces of time-series data that indicates nucleic acid amplification reaction occurring in the respective storage parts 22. On the basis of the time-series data, the controller 401 determines whether or not the substance to be detected is contained in each storage part 22, and causes the display unit 402 to display a determination result and the like. Then, the nucleic acid analysis ends.

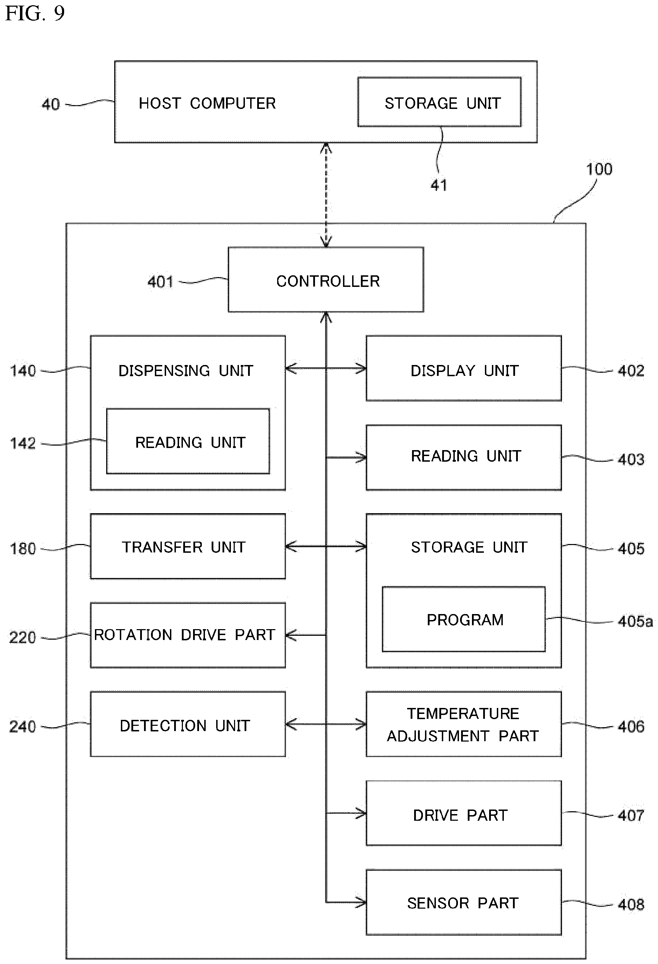

As shown in FIG. 9, the nucleic acid analysis device 100 includes the dispensing unit 140, the transfer unit 180, the rotation drive unit 220, the detection unit 240, the controller 401, the display unit 402, and the reading unit 403, as described above. In addition, the nucleic acid analysis device 100 includes a storage unit 405, a temperature adjustment part 406, a drive part 407, and a sensor part 408.

The controller 401 is implemented as a CPU or a microcomputer. The controller 401 may be implemented by a CPU and a microcomputer. The controller 401 controls components of the nucleic acid analysis device 100, and receives signals from the components of the nucleic acid analysis device 100. Upon receiving a start instruction from the operator through the display unit 402, the controller 401 starts processing of a sample, i.e., a nucleic acid analysis process. On the basis of electric signals of fluorescence detected by the detection unit 240, the controller 401 generates a plurality of pieces of time-series data that indicates nucleic acid amplification reaction occurring in the respective storage parts 22 of the amplification container 20. On the basis of the generated time-series data, the controller 401 determines "positive" or "negative" with respect to the detection target nucleic acid in which the detection target site of the nucleic acid has mutated.

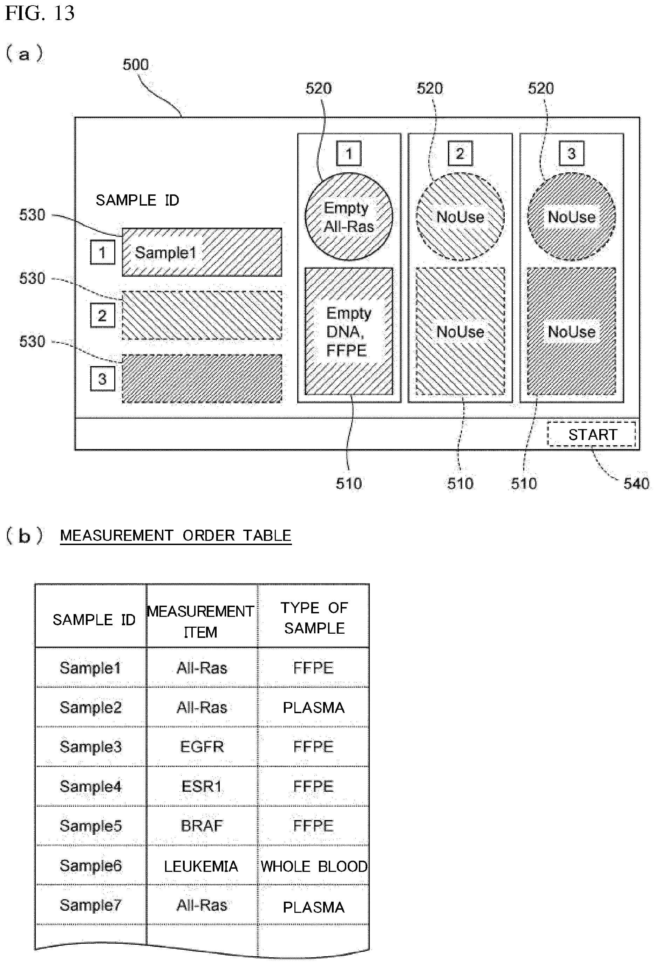

The controller 401 is communicably connected to a host computer 40. The host computer 40 includes a storage unit 41. The storage unit 41 stores therein a measurement order table in which a measurement order for each sample is registered. The measurement order table will be described later with reference to FIG. 13(b).

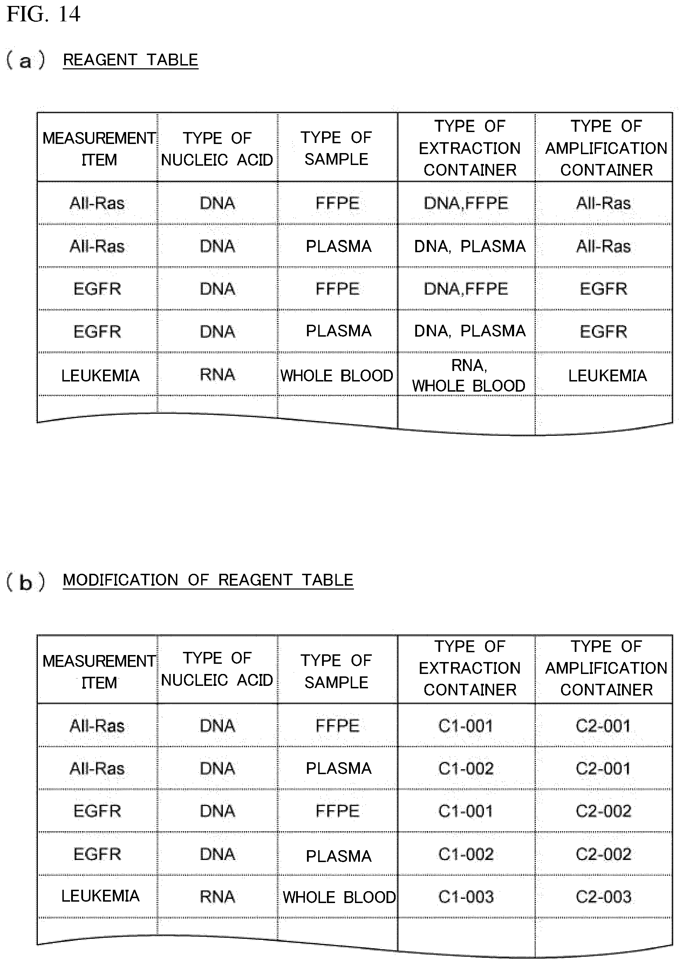

The storage unit 405 is implemented by a RAM, a ROM, a hard disk, and the like. The storage unit 405 stores therein in advance a program 405a for performing nucleic acid analysis. The storage unit 405 stores therein a reagent table for linking a measurement item, the type of nucleic acid, and the type of the sample, to the type of the extraction container 10 and the type of the amplification container 20. The reagent table will be described later with reference to FIG. 14(a). In addition, the storage unit 405 stores results of nucleic acid analyses.

The temperature adjustment part 406 includes the temperature adjustment parts 150, 160, 230. The drive part 407 includes various drive parts provided in the nucleic acid analysis device 100. The sensor part 408 includes the sensor 404, the first sensor 112a, the second sensor 122, and various sensors provided in the nucleic acid analysis device 100.

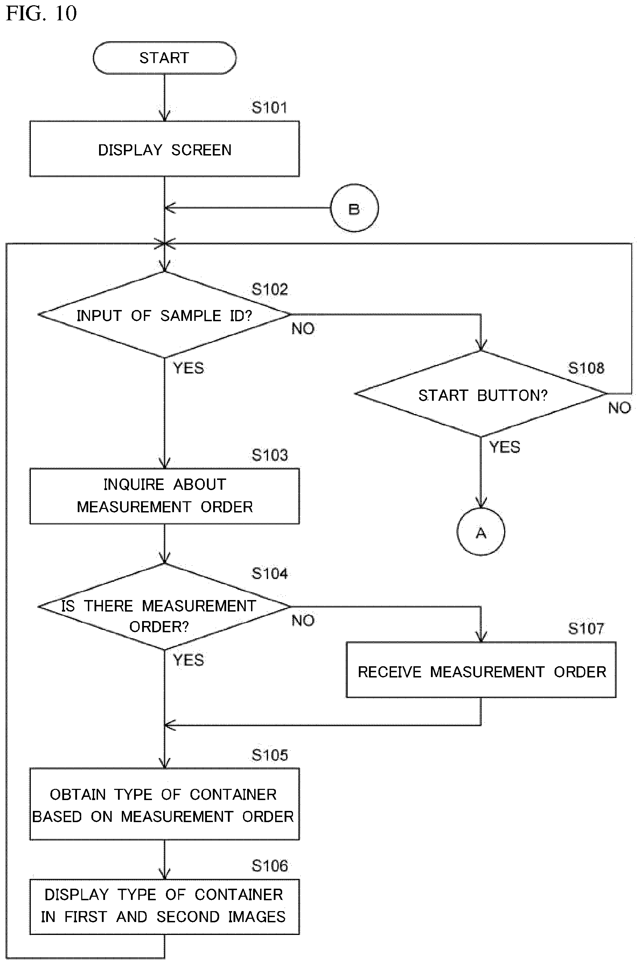

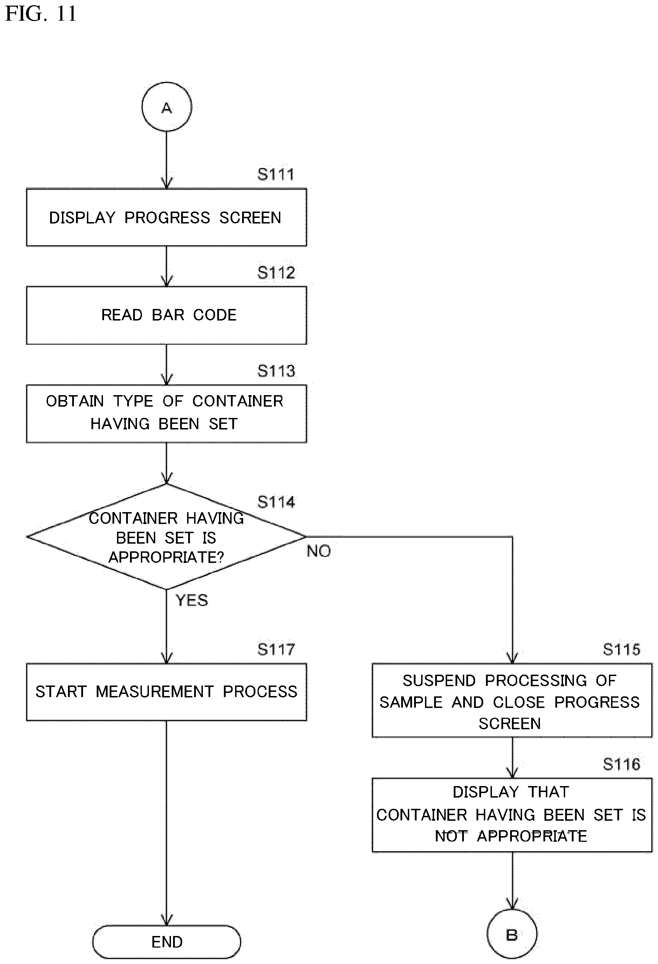

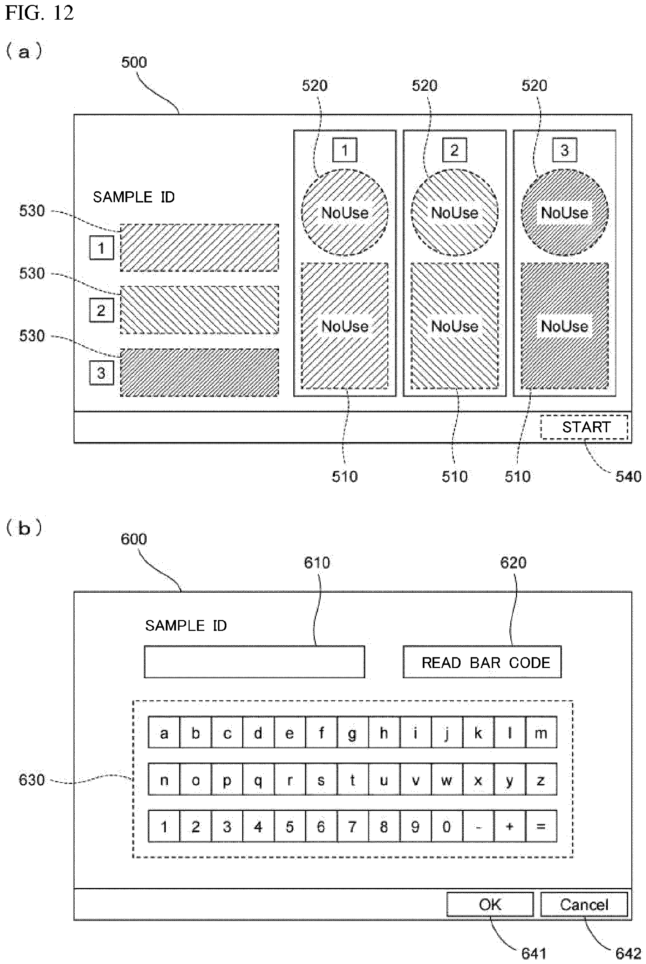

Next, with reference to FIGS. 10, 11, out of processes performed by the nucleic acid analysis device 100, a process up to the start of a measurement process is described. The controller 401 performs the process shown in FIGS. 10, 11 by executing the program 405a. In the following description, FIG. 12(a) to FIG. 17(b) are referred to as appropriate.

As shown in FIG. 10, in step S101, when an operator has performed an operation of causing the screen 500 to be displayed, the controller 401 causes the display unit 402 to display the screen 500 shown in FIG. 12(a). Subsequently, the controller 401 waits until a sample ID is inputted by the operator in step S102, or until the start button 540 is touched by the operator in step S108.

As shown in FIG. 12(a), the screen 500 has a configuration similar to that of the screen 500 shown in FIG. 1(a). When no sample ID has been inputted, the display regions 530 are blank as shown in FIG. 12(a), and "No Use" is displayed in the first images 510 and the second images 520. When inputting a sample ID, the operator touches, on the screen 500, a first image 510 or a display region 530 that corresponds to the lane in which the sample is to be set. Accordingly, as shown in FIG. 12(b), an input screen 600, instead of the screen 500, is displayed on the display unit 402.

The touch operation onto various portions of the screen displayed on the display unit 402 is realized by the operator touching a surface portion of the display unit 402. In a case where a display unit for displaying a screen and an input unit for performing an input are separately provided, the operator performs an operation of clicking or the like onto various portions of the screen, by operating the input unit while referring to the screen displayed on the display unit.

As shown in FIG. 12(b), the input screen 600 includes an input region 610, a bar code reading button 620, an input button region 630, an OK button 641, and a cancel button 642. Inputting of a sample ID to the input region 610 is performed through the bar code reading button 620 or the buttons in the input button region 630. When the bar code reading button 620 is used, the operator touches the bar code reading button 620 to bring the reading unit 403 close to the sample container storing the sample, whereby the sample ID is read by the reading unit 403 from the bar code label of the sample container. The sample ID read by the reading unit 403 is displayed in the input region 610. When the buttons in the input button region 630 are used, the operator touches buttons in the input button region 630, to input a sample ID. The sample ID inputted through the buttons in the input button region 630 is also displayed in the input region 610.

When the OK button 641 has been pushed by the operator, the controller 401 determines that a sample ID has been inputted in step S102, and advances the process to step S103. At this time, the controller 401 closes the input screen 600, and causes the display unit 402 to display the screen 500 again. Then, as shown in FIG. 13(a), the controller 401 causes the sample ID inputted through the input screen 600, to be displayed in the display region 530. In the example shown in FIG. 13(a), "Sample 1" is displayed as the sample ID in the display region 530 corresponding to the first lane. Meanwhile, when the cancel button 642 has been pushed by the operator, the controller 401 determines that no sample ID has been inputted in step S102, and advances the process to step S108. At this time, the controller 401 discards the sample ID inputted through the input screen 600, closes the input screen 600, and causes the display unit 402 to display the screen 500 again.

In step S103, on the basis of the sample ID inputted through the input screen 600, the controller 401 inquires about a measurement order to the host computer 40.

As shown in FIG. 13(b), the measurement order table includes, for each measurement order, a sample ID, a measurement item, and the type of the sample, as the items. Each measurement order is registered in the measurement order table in advance before nucleic acid analysis is performed in the nucleic acid analysis device 100.

Upon receiving the inquiry about the measurement order from the nucleic acid analysis device 100, the host computer 40 refers to the measurement order table stored in the storage unit 41, and performs a process of extracting a measurement order on the basis of the sample ID included in the content of the inquiry. When the inquired measurement order having the sample ID is stored, the host computer 40 transmits the measurement order having the sample ID, to the controller 401. When the inquired measurement order having the sample ID is not stored, the host computer 40 transmits a notification that the measurement order having the sample ID is not present, to the controller 401. Upon receiving, from the host computer 40, a result of the inquiry made in step S103, the controller 401 advances the process to step S104.

In step S104, on the basis of the result received from the host computer 40, the controller 401 determines whether the measurement order is present or not. When the measurement order is included in the received result, the controller 401 advances the process from step S104 to step S105.

The measurement order table may not necessarily be stored in the storage unit 41 of the host computer 40, and may be stored in the storage unit 405 of the nucleic acid analysis device 100. In this case, in step S103, the controller 401 refers to the measurement order table stored in the storage unit 405, and obtains the measurement order having the sample ID.

In step S105, the controller 401 refers to the reagent table on the basis of the measurement order, and obtains the types of the containers that should be set in the first image 510 and the second image 520.

As shown in FIG. 14(a), the reagent table includes, as the items, a measurement item, the type of nucleic acid to be extracted in the extraction container 10, the type of the sample set to the extraction container 10, the type of the extraction container 10, and the type of the amplification container 20. For each combination of a measurement item, the type of nucleic acid, and the type of the sample, the reagent table of Embodiment 1 stores the type of the extraction container 10 and the type of the amplification container 20.

More specifically, the type of nucleic acid is determined on the basis of the measurement item. For example, when the measurement item is "All-Ras" or "EGFR", the type of nucleic acid is "DNA". When the measurement item is "leukemia", the type of nucleic acid is "RNA". The type of the extraction container 10 is information discerned according to the type of nucleic acid and the type of the sample. That is, the type of the extraction container 10 is determined on the basis of the type of nucleic acid and the type of the sample. For example, when the type of nucleic acid is "DNA" and the type of the sample is "FFPE", the type of the extraction container 10 is "DNA, FFPE". Meanwhile, the type of the amplification container 20 is information discerned according to the measurement item. That is, the type of the amplification container 20 is determined on the basis of the measurement item. For example, when the measurement item is "All-Ras", the type of the amplification container 20 is "All-Ras".

For example, when the controller 401 has obtained the measurement order in the first line in FIG. 13(b), the controller 401 obtains "DNA, FFPE" as the type extraction container 10 and "All-Ras" as the type of the amplification container 20 from the reagent table in FIG. 14(a), on the basis of the measurement item "All-Ras" and the type of the sample "FFPE" included in this measurement order. When the controller 401 has obtained the measurement order in the sixth line in FIG. 13(b), the controller 401 obtains "RNA, whole blood" as the type of the extraction container 10 and "leukemia" as the type of the amplification container 20 from the reagent table in FIG. 14(a), on the basis of the measurement item "leukemia" and the type of the sample "whole blood" included in this measurement order.

The reagent table shown in FIG. 14(a) may be divided into: a table for associating the measurement item with the type of nucleic acid; a table for associating a combination of the type of nucleic acid and the type of the sample with the type of the extraction container 10; and a table for associating the measurement item with the amplification container 20. The type of the extraction container 10 may not necessarily be a character string composed of a character string indicating the type of nucleic acid and a character string indicating the type of the sample. The type of the amplification container 20 may not necessarily be a character string indicating the measurement item. For example, as shown in FIG. 14(b), the type of the extraction container 10 and the type of the amplification container 20 may be a reagent container name that allows discerning of the type of the extraction container 10 and a reagent container name that allows discerning of the type of the amplification container 20. Alternatively, the type of the extraction container 10 may be a character string for discerning the usage of the extraction container 10, and the type of the amplification container 20 may be a character string for discerning the usage of the amplification container 20.

The type of the extraction container 10 may be any information that is discerned according to the type of nucleic acid and the type of the sample at least, and may be, for example, information that is discerned according to the type of nucleic acid, the type of the sample, and the measurement item. The type of the amplification container 20 may be any information that is discerned according to the measurement item at least, and may be, for example, information that is discerned according to the type of nucleic acid, the type of the sample, and the measurement item.

The reagent table may not necessarily be stored in the storage unit 405 of the nucleic acid analysis device 100, and may be stored in the storage unit 41 of the host computer 40. In this case, in step S105, the controller 401 obtains the types of the containers based on the measurement order from the host computer 40.

In step S106, the controller 401 causes the types of the containers obtained in step S105 to be displayed in the first image 510 and the second image 520. For example, when a sample ID has been inputted for the first lane and the types of the containers have been obtained on the basis of this sample ID, the types of the containers are displayed in the first image 510 and the second image 520 of the first lane as shown in FIG. 13(a). In the example shown in FIG. 13(a), "DNA, FFPE" is displayed as the type of the extraction container 10 that should be set to the extraction container setting part 110 of the first lane, and "All-Ras" is displayed as the type of the amplification container 20 that should be set to the amplification container setting part 120 of the first lane.

In step S106, the character strings indicating the types of the containers as shown in FIG. 13(a) may not necessarily be displayed in the first image 510 and the second image 520, and any information that allows discerning of the types of the containers may be displayed therein. For example, as the character strings indicating the types of the containers, the reagent container names as shown in FIG. 14(b) may be displayed. Alternatively, diagrams indicating the types of the containers, character strings or diagrams for discerning the usages of the containers, or the like, may be displayed.

When the types of the containers that should be set are displayed in the first image 510 and the second image 520, the operator can understand the type of the extraction container 10 that should be set to the extraction container setting part 110, and the type of the amplification container 20 that should be set to the amplification container setting part 120. Accordingly, the operator can set containers of appropriate types to the respective container setting parts.

As shown as an example in the first lane in FIG. 13(a), when a sample ID has been inputted but containers have not been set in the corresponding extraction container setting part 110 and amplification container setting part 120, "Empty" is displayed in the first image 510 and the second image 520. The controller 401 detects that the extraction container 10 is not set to the extraction container setting part 110, on the basis of a result of detection by the first sensor 112a, and the controller 401 detects that the amplification container 20 is not set to the amplification container setting part 120, on the basis of a result of detection by the second sensor 122. When "Empty" is displayed, the color of the frame of each of the first image 510 and the second image 520 becomes a color different from that of the frame of "No Use". In FIG. 13(a), in order to indicate that the color of the frame has changed, the frame of each of the first image 510 and the second image 520 is indicated with a solid line, for convenience.

Meanwhile, when, as a result of the inquiry about the measurement order in step S103, the controller 401 has received a notification that the measurement order is not present, the controller 401 advances the process from step S104 to step S107. In step S107, the controller 401 receives a measurement order through the display unit 402.

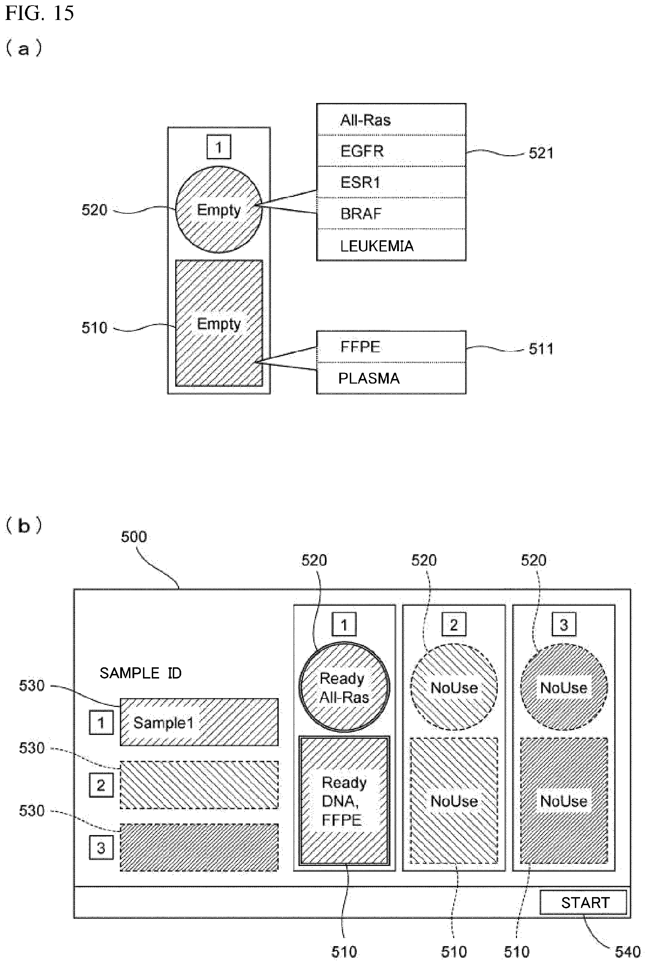

FIG. 15(a) shows a first operation portion 511 and a second operation portion 521 to be used when setting a measurement order for the first lane. The first operation portion 511 and the second operation portion 521 are each implemented in the form of a selection list.

By continuously touching the second image 520 for a predetermined time, the operator causes the second operation portion 521 to be displayed. Then, by touching an item in the second operation portion 521, the operator sets a measurement item for the first lane. Subsequently, by continuously touching the first image 510 for a predetermined time, the operator causes the first operation portion 511 to be displayed. The first operation portion 511 shown in FIG. 15(a) indicates a state where selectable items have been narrowed to two as a result of selection of "All-Ras" in the second operation portion 521. Then, the operator touches an item in the first operation portion 511, thereby setting the type of the sample for the first lane. The controller 401 receives the measurement order of the sample to be processed, through the first operation portion 511 and the second operation portion 521. Accordingly, even when a measurement order has not been set in advance, a measurement order can be easily set later.

The first operation portion 511 and the second operation portion 521 may not necessarily be selection lists, and may be any operation portion for receiving a measurement order of a sample to be processed. For example, the first operation portion 511 and the second operation portion 521 may be screens for respectively receiving a measurement item and the type of the sample. Alternatively, the first operation portion 511 may receive a measurement item, and the second operation portion 521 may receive the type of the sample.

Also in this case, in the subsequent step S105, the controller 401 obtains the types of the containers with reference to the reagent table, on the basis of the measurement order set in step S107. Then, in step S106, the controller 401 causes the obtained types of the containers to be displayed in the first image 510 and the second image 520. For example, when "All-Ras" has been set as the measurement item and "FFPE" has been set as the type of the sample through the first operation portion 511 and the second operation portion 521, the types of the containers are displayed in the first image 510 and the second image 520 of the first lane as illustrated in FIG. 13(a).

In a case of a form where the measurement order is not registered in advance, the processes of steps S103 and S104 are omitted. In this case, upon a sample ID being inputted, a measurement order is inputted in step S107, as described with reference to FIG. 15(a).

After performing the process of step S106, the controller 401 returns the process to step S102. Then, the controller 401 waits until a sample ID is inputted, or until the start button 540 is touched.

As shown in FIG. 13(a), when the types of the containers that should be set are displayed in the first image 510 and the second image 520, the operator confirms the displayed content of the first image 510 and the second image 520, opens the cover 100b, and sets the containers to the extraction container setting part 110 and the amplification container setting part 120. In the example shown in FIG. 13(a), "DNA, FFPE" is displayed in the first image 510 of the first lane. Thus, the operator sets an extraction container 10 of which the type is "DNA, FFPE", to the extraction container setting part 110 of the first lane. In addition, in the example shown in FIG. 13(a), "All-Ras" is displayed in the second image 520 of the first lane. Thus, the operator sets an amplification container 20 of which the type is "All-Ras" to the amplification container setting part 120 of the first lane.

As described above, when the operator sets an extraction container 10 to the extraction container setting part 110, the operator stores a sample of the type corresponding to the measurement order into the reaction part 11, and stores ethanol into the reagent storage part 15.

As a result of the containers being set to the extraction container setting part 110 and the amplification container setting part 120, "Ready" is displayed as shown in FIG. 15(b) in the first image 510 and the second image 520 where "Empty" has been displayed. The controller 401 detects that the extraction container 10 is set to the extraction container setting part 110, on the basis of the result of detection by the first sensor 112a, and the controller 401 detects that the amplification container 20 is set to the amplification container setting part 120, on the basis of the result of detection by the second sensor 122. When "Ready" is displayed, the color of the frame of each of the first image 510 and the second image 520 becomes a color different from those of the frames of "No Use" and "Empty". In FIG. 15(b), in order to indicate that the color of the frame has changed, the frame of each of the first image 510 and the second image 520 is indicated with a double solid line, for convenience.

"Empty" and "Ready" displayed in the first image 510 and the second image 520, and the color of the frame of each of the first image 510 and the second image 520, are each information regarding whether or not containers are set to the container setting parts. By referring to these pieces of information, the operator can understand whether or not a container is set in the corresponding container setting part. Thus, when the container is set, the operator can understand that it is not necessary to set a container to the container setting part, and when the container is not set, the operator can understand that it is necessary to set a container to the container setting part. Therefore, the operator can appropriately and smoothly set the extraction container 10 and the amplification container 20.

When the extraction container 10 and the amplification container 20 for the first lane are set, the cover 100b is closed, and thus, a state where the process can be started is established, the screen 500 enters a state shown in FIG. 15(b). On the basis of a result of detection by the sensor 404, the controller 401 detects that the cover 100b has been closed. When a state where the process can be started at at least one lane among the first to third lanes is established, the start button 540 is enabled. In FIG. 15(b), in order to indicate that the start button 540 has been enabled, the frame of the start button 540 is indicated with a solid line, for convenience.

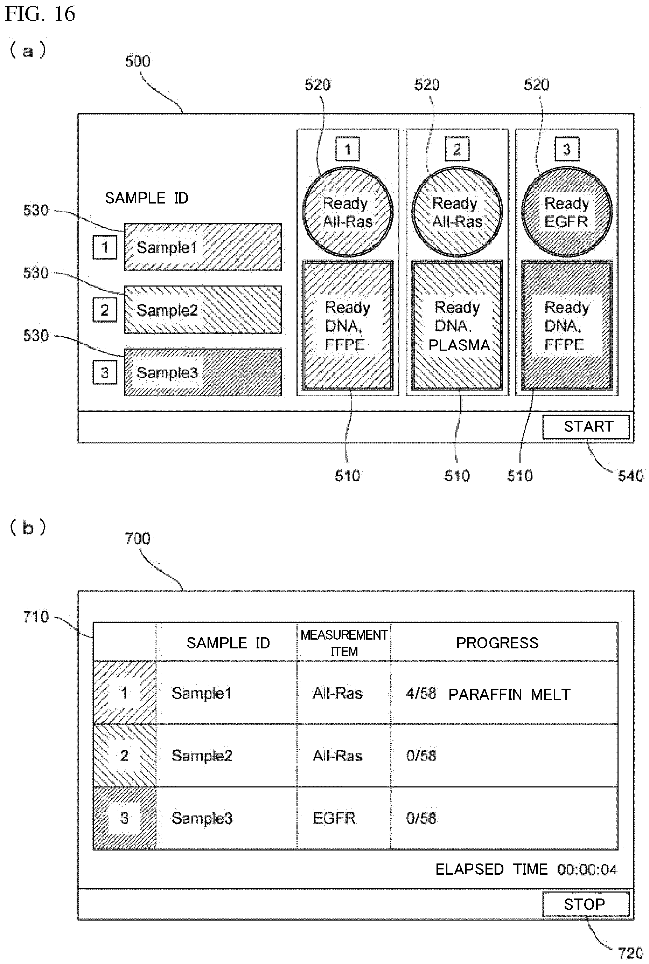

Further, if sample IDs have been inputted, and the extraction container 10 and the amplification container 20 have been set not only for the first lane but for the second and third lanes as well, and then the cover 100b is closed, the screen 500 enters a state shown in FIG. 16(a), for example. In the example shown in FIG. 16(a), the measurement orders in the second and third lines in FIG. 13(b) have been obtained on the basis of the sample IDs inputted for the second and third lanes. Accordingly, "DNA, plasma" and "All-Ras" are respectively displayed in the first image 510 and the second image 520 of the second lane, and "DNA, FFPE" and "EGFR" are respectively displayed in the first image 510 and the second image 520 of the third lane. The operator sets the extraction container 10 and the amplification container 20 in accordance with the type of the containers shown in the first image 510 and the second image 520, also for the second and third lanes. Accordingly, the screen 500 enters the state shown in FIG. 16(a).

In Embodiment 1, the first image 510 and the second image 520 of each lane are associated with the display region 530 corresponding to the lane. Thus, the operator can easily understand the correspondence relationship between the first image 510 and the second image 520, and the sample ID. Therefore, for example, the operator can easily understand to which lane's container setting parts the operator should set the extraction container 10 and the amplification container 20 of the types that should be used in the processing of the sample indicated by each sample ID. Thus, the operator can appropriately set the extraction container 10 and the amplification container 20 of the types that are appropriate for the sample indicated by each sample ID, to the extraction container setting part 110 and the amplification container setting part 120 associated with the sample ID.

The first image 510 and the second image 520 of one lane and the display region 530 corresponding to the lane are associated with each other by the same color and the same lane number. Accordingly, the operator can more intuitively understand the correspondence relationship between the first image 510 and the second image 520, and the sample ID of each lane, by referring to the color of the first image 510 and the second image 520 or the lane number displayed in the vicinity of the first image 510 and the second image 520. Thus, the operator can more smoothly and appropriately set the containers to the respective container setting parts. The first image 510 and the second image 520 of one lane, and the display region 530 corresponding to the lane, may be linked to each other by means of a character or a diagram.

The extraction container setting part 110 and the amplification container setting part 120 of one lane, and the first image 510 and the second image 520 corresponding to the lane are provided with the same color. Accordingly, the operator can intuitively associate, by means of the color, the extraction container setting part 110, the amplification container setting part 120, the first image 510, and the second image 520 of the same lane, with one another. Thus, the operator can more smoothly and appropriately set the containers to the respective container setting parts, while referring to the images displayed on the display unit 402.

The extraction container setting part 110 and the amplification container setting part 120 of the same lane are disposed so as to be arranged in the depth direction, i.e., the X axis direction, of the device body. The extraction container setting parts 110 and the amplification container setting parts 120 of different lanes are arranged in the width direction, i.e., the Y-axis direction, of the device body. Accordingly, the width of the device body can be reduced, and thus, the nucleic acid analysis device 100 can be smoothly set on a desk, an installation table, or the like.

In addition to the above-described configurations of the extraction container setting part 110 and the amplification container setting part 120, the display unit 402 is disposed at the front face of the device body, the first image 510 and the second image 520 corresponding to the same lane are arranged in the screen-vertical direction, and the first images 510 and the second images 520 of different lanes are arranged in the screen-horizontal direction. Accordingly, when viewed from the front side of the device body, the layout of the first images 510 and the second images 520 displayed on the display unit 402, and the layout of the extraction container setting parts 110 and the amplification container setting parts 120 in the device body match each other. Accordingly, in the operation of setting the containers to the device body, the operator can more intuitively associate the first image 510 and the second image 520 with the respective container setting parts. Therefore, the operator can easily set the containers to the respective container setting parts, while referring to the first image 510 and the second image 520.

When the start button 540 has been pushed by the operator after the start button 540 has been enabled, the controller 401 advances the process from step S108 in FIG. 10 to step S111 in FIG. 11, thereby starting a process on the sample to be processed.

As shown in FIG. 11, in step S111, the controller 401 causes the display unit 402 to display a progress screen 700 shown in FIG. 16(b). As shown in FIG. 16(b), the progress screen 700 includes a progress list 710 and a stop button 720. The progress list 710 indicates the progress statuses of the processing of the samples being performed in the first to third lanes. In each of the display rows of the first to third lanes, the progress list 710 includes a sample ID, a measurement item, and a progress status, as the items. By touching the stop button 720, the operator can stop all the processes being performed in the nucleic acid analysis device 100.

Subsequently, in step S112, the controller 401 reads bar codes from the bar code label 17 of the extraction container 10 and the bar code label 28 of the amplification container 20 set in each lane. Specifically, the controller 401 drives the dispensing unit 140 to move the reading unit 142 to directly above the bar code label 17, 28, and drives the reading unit 142 to read the bar code from the bar code label 17, 28. In step S113, from the information included in the bar code, the controller 401 obtains the type of the extraction container 10 set to the extraction container setting part 110 of each lane, and the type of the amplification container 20 set to the amplification container setting part 120 of each lane.

In step S114, on the basis of the measurement order of the sample to be processed and the types of the containers read by the reading unit 142, the controller 401 determines whether or not the extraction container 10 set to the extraction container setting part 110 and the amplification container 20 set to the amplification container setting part 120 are appropriate.

Specifically, the controller 401 determines whether or not the type of the extraction container 10 obtained from the bar code of the extraction container 10 set in each lane matches the type of the extraction container 10 that should be set to the extraction container setting part 110 shown in the first image 510 of the corresponding lane. In addition, the controller 401 determines whether or not the type of the amplification container 20 obtained from the bar code of the amplification container 20 set in each lane matches the type of the amplification container 20 that should be set to the amplification container setting part 120 shown in the second image 520 of the corresponding lane. When the type of the container obtained from the bar code and the type of the container that should be set match each other, the controller 401 determines that an appropriate container is set to the target container setting part.

With respect to all of the extraction containers 10 and the amplification containers 20, the controller 401 performs the determination as to whether or not the type of the extraction container 10 obtained from the bar code and the type of the extraction container 10 shown in the first image 510 match each other, and the determination as to whether or not the type of the amplification container 20 obtained from the bar code and the type of the amplification container 20 shown in the second image 520 match each other.

When at least one container that is not appropriately set is included among all of the extraction containers 10 and the amplification containers 20, the controller 401 advances the process from step S114 to step S115. In step S115, the controller 401 stops the processes on the samples started by the start button 540 being pushed, and closes the progress screen 700. Then, in step S116, the controller 401 causes the display unit 402 to display the screen 500 again, and indicates that setting of the container has been inappropriate, in the first image 510 or the second image 520 corresponding to the container that has been determined, in step S114, not to be appropriately set.

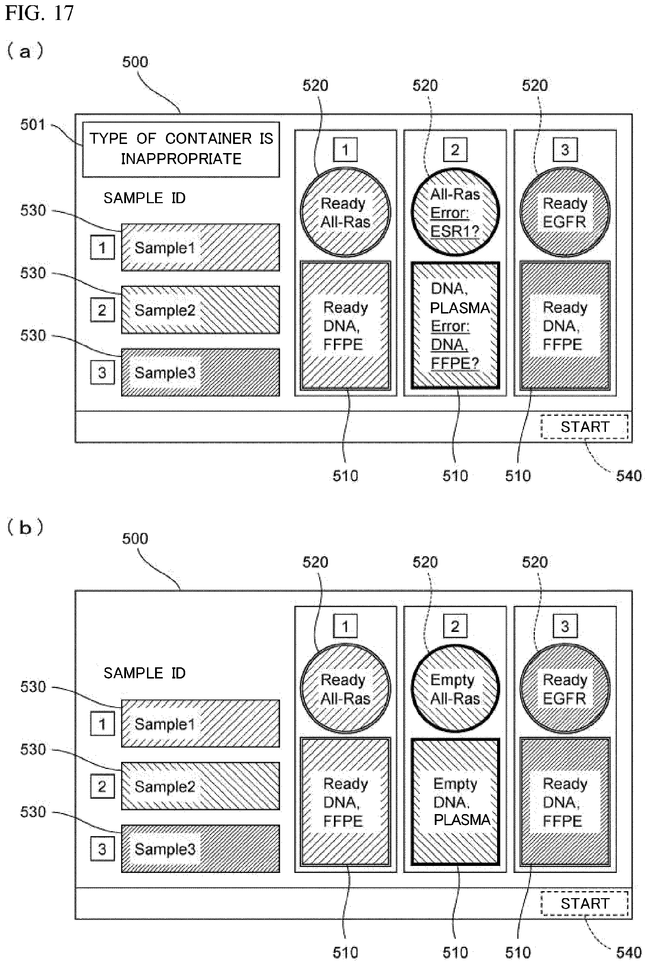

FIG. 17(a) illustrates the screen 500 at the time when containers having been set are not appropriate. FIG. 17(a) shows a case where the extraction container 10 and the amplification container 20 set in the second lane are not appropriate. In this case, errors are displayed in the first image 510 and the second image 520 of the second lane. Specifically, since the type of the extraction container 10 obtained from the bar code of the extraction container 10 set to the extraction container setting part 110 of the second lane is "DNA, FFPE", "Error: DNA, FFPE?" is displayed in the first image 510 of the second lane. In addition, since the type of the amplification container 20 obtained from the bar code of the amplification container 20 set in the amplification container setting part 120 of the second lane is "ESR1", "Error: ESR1?" is displayed in the second image 520 of the second lane.

In the example shown in FIG. 17(a), "DNA, plasma" and "All-Ras" are respectively displayed as the appropriate types of the containers, in the first image 510 and the second image 520 of the second lane.

In this case, the color of the frame of each of the first image 510 and the second image 520 in the second lane becomes red. In FIG. 17(a), in order to indicate that the color of the frame is red, the frame of each of the first image 510 and the second image 520 is indicated with a thick solid line, for convenience. Further, an error label 501 indicating that a container of an inappropriate type has been set is displayed on the screen 500. For example, a character string "the type of the container is inappropriate" is displayed in the error label 501. The error label 501 may indicate which container of which lane is inappropriate.

In this manner, the operator can visually recognize that the set container is inappropriate, by referring to the displays in the first image 510 and the second image 520, the color of the frame of each of the first image 510 and the second image 520, the error label 501, and the like. Accordingly, the operator can prevent the processing of the sample from being performed by use of a container of an inappropriate type. Therefore, by subsequently setting an appropriate container, it is possible to perform an appropriate process on the sample. In addition, the operator can smoothly replace the wrong container having been set, with a container of an appropriate type.

When the error as described above is displayed, the process on the sample is stopped. Thus, the processing of the sample by use of a container of an inappropriate type can be assuredly prevented from being performed. Therefore, by subsequently setting an appropriate container, it becomes possible to perform an appropriate process on the sample. In addition, it is possible to prevent the aluminium seal 10a from being opened in the wrong extraction container 10 thereby wasting the extraction container 10, and it is possible to prevent an extract from being injected into a wrong amplification container 20 thereby wasting the amplification container 20.

When an inappropriate container is set as described above, the start button 540 is disabled so as not to be operated, as shown in FIG. 17(a). In FIG. 17(a), in order to indicate that the start button 540 has been disabled, the frame of the start button 540 is indicated with a broken line, for convenience. Accordingly, the process can be assuredly prevented from being performed with the inappropriate container being set.

In a case where a display for associating the display region 530 with the first image 510 and the second image 520 is omitted, the display region 530 may be connected to the first image 510 and the second image 520 by means of lines on the screen 500, so as to indicate for which sample ID an inappropriate container has been set.

After step S116 in FIG. 11 has been performed, the controller 401 returns the process to step S102 in FIG. 10. In this case, the operator opens the cover 100b, and removes the containers from the extraction container setting part 110 and the amplification container setting part 120 where the errors have occurred. When the errors as shown in FIG. 17(a) have been displayed, the operator removes the extraction container 10 and the amplification container 20 of the second lane. Accordingly, as shown in FIG. 17(b), the display of the error label 501 disappears, and "Empty" is displayed in each of the first image 510 and the second image 520 of the second lane. Even after the inappropriate containers have been removed, the color of the frame of each of the first image 510 and the second image 520 where the inappropriate containers have been set is maintained to be red, as shown in FIG. 17(b). Accordingly, the operator can understand that appropriate containers have not yet been set.

Then, the operator sets appropriate containers to the container setting parts where the containers have been removed. At this time, the controller 401 detects that: the inappropriate containers have been removed; and then containers have been set. Accordingly, as shown in FIG. 16(a), the frame of each of the first image 510 and the second image 520 is indicated with a double line, and "Ready" is displayed in the first image 510 and the second image 520. Then, as shown in FIG. 16(a), when the operator closes the cover 100b in a state where the error display has disappeared, the start button 540 becomes enabled again.

Then, the operator touches the start button 540 to start the process again. Accordingly, the controller 401 advances the process from step S108 in FIG. 10 to step S111 in FIG. 11, thereby starting again the process on each sample to be processed.

Also when the process has been started again, the controller 401 causes the progress screen 700 to be displayed in step S111, reads bar codes from all of the extraction containers 10 and the amplification containers 20 in step S112, and obtains the types of the extraction container 10 and the amplification container 20 set in each lane in step S113. Then, in step S114, on the basis of the obtained types of the extraction containers 10 and the amplification containers 20, the controller 401 determines again whether or not appropriate containers have been set.

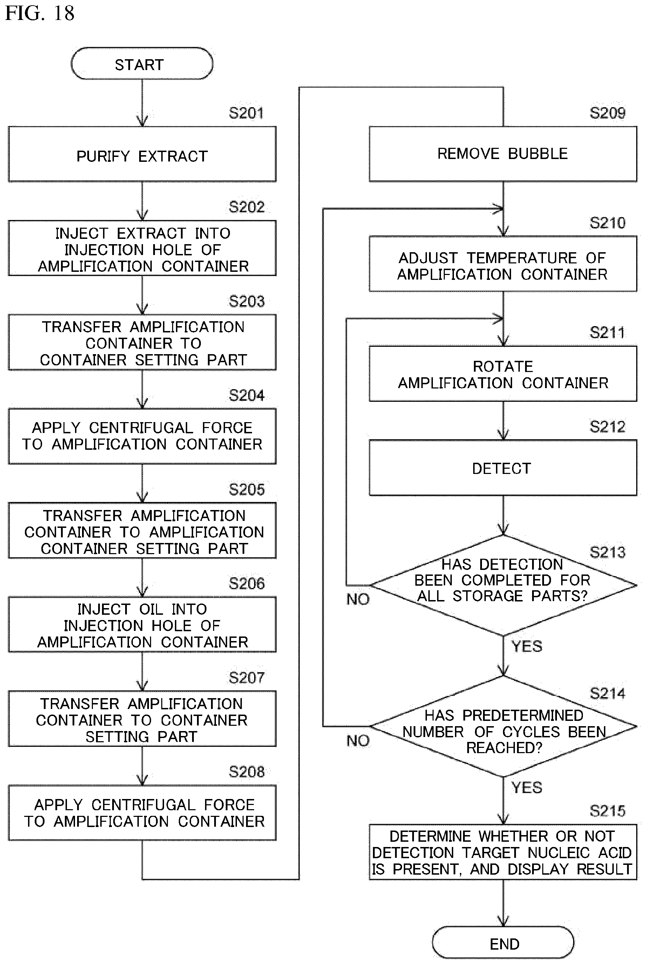

When having determined in step S114 that appropriate containers have been set, the controller 401 advances the process from step S114 to step S117. In step S117, the controller 401 starts a measurement process for each lane for which a sample ID has been inputted and the extraction container 10 and the amplification container 20 have been set. Accordingly, a process shown in FIG. 18 is performed for each sample to be processed.

Next, with reference to FIG. 18, out of the processes performed by the nucleic acid analysis device 100, the measurement process is described. The controller 401 performs the process shown in FIG. 18 by executing the program 405a. The controller 401 performs, in parallel, a plurality of the measurement processes started in step S117 in FIG. 10. In the following, a measurement process started for one lane is described.

Upon start of the measurement process, the controller 401 drives the dispensing unit 140 to attach a puncture tip 31 to the lower end of the nozzle 141. The controller 401 drives the dispensing unit 140 to pierce the aluminium seal 10a with the puncture tip 31, thereby opening the upper portions of the reagent storage part 12, the reagent storage parts 13a to 13h, and the waste liquid storage part 16 of the extraction container 10. In step S201, the controller 401 drives the dispensing unit 140 to purify the extract in the extraction container 10. In the purification of the extract, a pipette tip 32 is attached and replaced as appropriate with respect to the nozzle 141, and a liquid is suctioned and discharged through the pipette tip 32 by the nozzle 141.

In step S201, specifically, the controller 401 performs the control described below. In the following, the control performed in step S201 is described using an example case where: the type of the sample is a FFPE section; and the type of nucleic acid to be extracted is DNA.

The controller 401 causes a solubilizing liquid in the reagent storage part 13a to be dispensed into the reaction part 11. Accordingly, the FFPE section is immersed. The controller 401 causes the temperature adjustment part 150 to move upward, to heat the reaction part 11 by the heater 152. Accordingly, the paraffin melts.

Next, the controller 401 causes the proteinase K in the reagent storage part 13b to be dispensed into the reaction part 11, and causes the oil in the reagent storage part 13c to be dispensed into the reaction part 11. The oil in the reagent storage part 13c is mineral oil. Subsequently, the controller 401 adjusts the temperature of the reaction part 11 by means of the temperature adjustment part 150. Accordingly, protein in the reaction part 11 is degraded, and nucleic acid is extracted from cells.

Next, the controller 401 causes the magnetic force application part 170 to be close to the reagent storage part 12. Accordingly, magnetic particles in the reagent storage part 12 are collected to the wall surface of the reagent storage part 12. Then, the controller 401 drives the dispensing unit 140 to transfer the magnetic particle preservative liquid in the reagent storage part 12 to the waste liquid storage part 16. Then, the controller 401 causes the magnetic force application part 170 to be away from the reagent storage part 12. Subsequently, the controller 401 drives the dispensing unit 140 to dispense the ethanol in the reagent storage part 15 and the reagent for extraction in the reagent storage part 13e, into the mixing part 14c, and dispense the mixture of the ethanol and the reagent for extraction stored in the mixing part 14c into the reagent storage part 12.

Subsequently, the controller 401 drives the dispensing unit 140 to move the specimen solution in the reaction part 11 to the reagent storage part 12, and repeat suction and discharge in the reagent storage part 12, thereby stirring the specimen solution in the reagent storage part 12. Subsequently, the controller 401 drives the temperature adjustment part 160 to adjust the temperature of the reagent storage part 12. Accordingly, nucleic acid is captured by the magnetic particles. Subsequently, the controller 401 causes the magnetic force application part 170 to be close to the reagent storage part 12. Accordingly, the magnetic particles in the reagent storage part 12 are collected to the wall surface of the reagent storage part 12. Then, the controller 401 drives the dispensing unit 140 to suction the supernatant in the reagent storage part 12, and transfer the suctioned liquid to the waste liquid storage part 16. Then, the controller 401 causes the magnetic force application part 170 to be away from the reagent storage part 12.

Next, the controller 401 drives the dispensing unit 140 to dispense the ethanol in the reagent storage part 15 and the stock solution of the first washing liquid in the reagent storage part 13h, into the mixing part 14b, and dispense the mixture of the ethanol and the first washing liquid stored in the mixing part 14b into the reagent storage part 12. Subsequently, the controller 401 drives the dispensing unit 140 to stir the specimen solution in the reagent storage part 12. Subsequently, the controller 401 causes the magnetic force application part 170 to be close to the reagent storage part 12. Then, the controller 401 controls the dispensing unit 140 to suction the supernatant in the reagent storage part 12, and transfer the suctioned liquid to the waste liquid storage part 16. Then, the controller 401 causes the magnetic force application part 170 to be away from the reagent storage part 12.

Similarly, the controller 401 drives the dispensing unit 140 to dispense the ethanol in the reagent storage part 15 and the stock solution of the second washing liquid in the reagent storage part 13f, into the mixing part 14d, and dispense the mixture of the ethanol and the second washing liquid stored in the mixing part 14d into the reagent storage part 12. Subsequently, the controller 401 drives the dispensing unit 140 to stir the specimen solution in the reagent storage part 12. Subsequently, the controller 401 causes the magnetic force application part 170 to be close to the reagent storage part 12. Then, the controller 401 drives the dispensing unit 140 to suction the supernatant in the reagent storage part 12, and transfer the suctioned liquid into the waste liquid storage part 16. Then, the controller 401 causes the magnetic force application part 170 to be away from the reagent storage part 12. In this manner, impurities in the reagent storage part 12 are washed.

Next, the controller 401 drives the dispensing unit 140 to dispense the elution liquid in the reagent storage part 13d into the reagent storage part 12, and stir the specimen solution in the reagent storage part 12. Subsequently, the controller 401 drives the temperature adjustment part 160 to adjust the temperature of the reagent storage part 12. Accordingly, nucleic acid in the reagent storage part 12 elutes from the magnetic particles.

Next, the controller 401 causes the magnetic force application part 170 to be close to the reagent storage part 12. Accordingly, the magnetic particles in the reagent storage part 12 are collected to the wall surface of the reagent storage part 12. Subsequently, the controller 401 drives the dispensing unit 140 to transfer the specimen solution in the reagent storage part 12 to the mixing part 14a. Then, the controller 401 causes the magnetic force application part 170 to be away from the reagent storage part 12. Subsequently, the controller 401 causes the stock solution of the diluent in the reagent storage part 13g to be dispensed into the mixing part 14a, to stir the specimen solution in the mixing part 14a. Accordingly, the concentration of the specimen in the mixing part 14a is adjusted, whereby an extract is completed in the mixing part 14a.

When the type of the sample is plasma, and the type of nucleic acid to be extracted is DNA, the step of deparaffinization by the heat treatment is omitted, when compared with the above-described process. When the type of the sample is whole blood, and the type of nucleic acid to be extracted is RNA, the step of deparaffinization by the heat treatment and the proteinase K reaction step are omitted, when compared with the above-described process. In this manner, the step of purifying the extract in step S201 is performed in accordance with the type of the sample and the type of nucleic acid.

In step S202, the controller 401 drives the dispensing unit 140 to inject the extract in the mixing part 14a into the injection hole 21 of the amplification container 20 set to the amplification container setting part 120. In step S203, the controller 401 drives the transfer unit 180 to transfer, to the container setting part 210, the amplification container 20 set to the amplification container setting part 120. In step S204, the controller 401 drives the rotation drive unit 220 to rotate the amplification container 20 at a high speed, to apply centrifugal force to the amplification container 20. Accordingly, the extract injected into the injection hole 21 is sent through the flow paths 23 to the storage parts 22.

In step S205, the controller 401 drives the transfer unit 180 to transfer the amplification container 20 rotated by the rotation drive unit 220, to the amplification container setting part 120. In step S206, the controller 401 drives the dispensing unit 140 to inject the oil in the reagent storage part 13c into the injection hole 21 of the amplification container 20 rotated by the rotation drive unit 220 and transferred by the amplification container setting part 120. The oil to be used in step S206 may be stored in advance in a predetermined storage part in the nucleic acid analysis device 100.

Subsequently, in step S207, the controller 401 drives the transfer unit 180 to transfer, to the container setting part 210, the amplification container 20 into which the oil has been injected. In step S208, the controller 401 drives the rotation drive unit 220 to rotate the amplification container 20 at a high speed, to apply centrifugal force to the amplification container 20. Accordingly, air in the flow paths 23 of the amplification container 20 is replaced with the oil injected from the injection hole 21.

Next, in step S209 to S215, the controller 401 performs detection of nucleic acid amplification reaction and nucleic acid analysis. In Embodiment 1, the controller 401 performs detection and analysis on the basis of the principle of BNA Clamp PCR. The principle of detection and analysis is not limited to that of BNA Clamp PCR, and may be the principle of PCR+Invader, for example.

In step S209, the controller 401 causes bubbles in the amplification container 20 to be removed through the injection hole 21. Specifically, the controller 401 drives the temperature adjustment part 230 to heat the amplification container 20, and drives the rotation drive unit 220 to rotate the amplification container 20 at a high speed. Accordingly, centrifugal force is applied to the amplification container 20, and bubbles in the amplification container 20 are removed through the injection hole 21.

In step S210, the controller 401 drives the temperature adjustment part 230 to adjust the temperature of the amplification container 20. Subsequently, in step S211, the controller 401 drives the rotation drive unit 220 to rotate the amplification container 20 such that a storage part 22 is positioned at the detection position of the detection unit 240. In step S212, the controller 401 drives the detection unit 240 to detect nucleic acid amplification reaction occurring in the storage part 22. Specifically, the detection unit 240 applies excitation light to the storage part 22, and receives fluorescence generated from the storage part 22, by the light detector 242d. On the basis of an electric signal outputted by the light detector 242d, the controller 401 obtains a fluorescence intensity, and stores the obtained fluorescence intensity into the storage unit 405.

In step S213, the controller 401 determines whether or not the detection has been completed for all of the storage parts 22. When the detection has not been completed for all of the storage parts 22, the controller 401 returns the process to step S211. In this case, in step S211, the controller 401 drives the rotation drive unit 220 to rotate the amplification container 20 only by the pitch in the circumferential direction of the storage parts 22, such that the adjacent storage part 22 for which the detection has not been completed is positioned at the detection position. Then, as described above, in step S212, detection of nucleic acid amplification reaction is performed. In this manner, the operation performed by the rotation drive unit 220 of rotating the amplification container 20 by the pitch in the circumferential direction of the storage parts 22, and the operation performed by the detection unit 240 of detecting nucleic acid amplification reaction in the storage part 22, are repeated. Then, nucleic acid amplification reaction is sequentially detected from all of the storage parts 22 arranged in the circumferential direction.

When the detection has been completed for all of the storage parts 22, the controller 401 determines, in step S214, whether or not the number of cycles has reached a predetermined number of cycles. Here, the cycle denotes the process performed from step S210 to S213. The predetermined number of cycles is 55, for example. That is, in step S214, it is determined whether or not one cycle composed of step S210 to S213 has been performed the predetermined number of cycles in total. When the number of cycles has not reached the predetermined number of cycles, the controller 401 returns the process to step S210. Then, the controller 401 performs the cycle composed of step S210 to S213 again.

When the number of cycles has reached the predetermined number of cycles, the controller 401 determines, in step S215, whether or not the detection target nucleic acid is present in each storage part 22, and causes the display unit 402 to display a determination result and the like. In this manner, the process of nucleic acid analysis for one sample ends. When the process of nucleic acid analysis for one sample ends, the controller 401 drives the transfer unit 180 to transfer, to the amplification container setting part 120, the amplification container 20 set to the container setting part 210. The transferred amplification container 20 is then discarded.

### <Modification 1 of Embodiment 1>

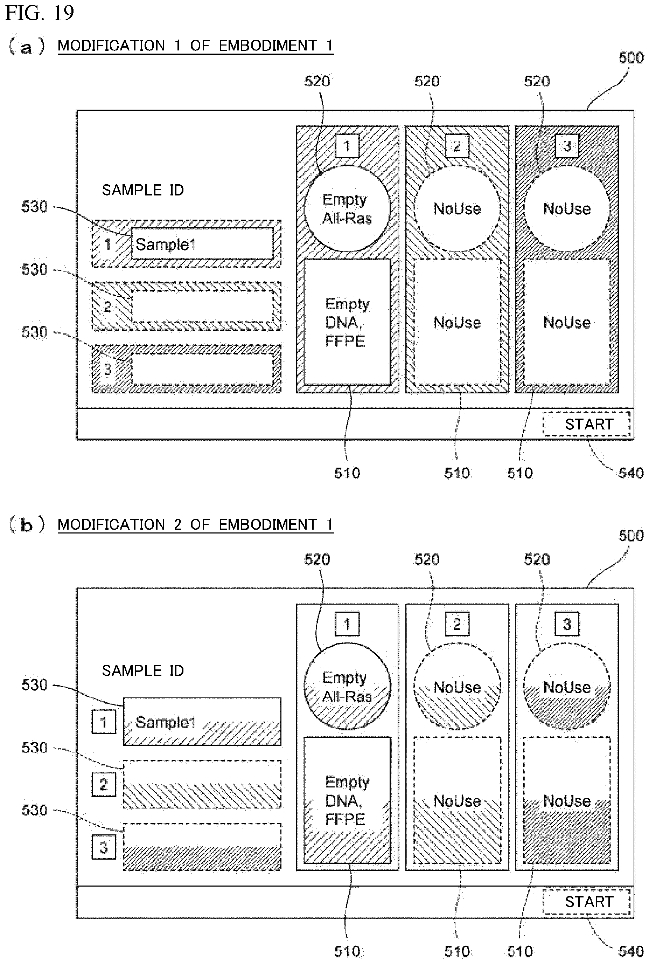

In Modification 1 of Embodiment 1, the region to be provided with a color in the screen 500 is changed when compared with Embodiment 1. As shown in FIG. 19(a), in Modification 1 of Embodiment 1, within a rectangular region surrounding the first image 510 and the second image 520, a region outside the first image 510 and the second image 520 is provided with a color. The color provided to the outside region of the first image 510 and the second image 520 corresponding to each lane is the same as that in Embodiment 1. In addition, within a rectangular region surrounding each display region 530, a region outside the display region 530 is provided with a color. The color provided to the outside region of the display region 530 corresponding to each lane is the same as that in Embodiment 1. The other configurations in Modification 1 of Embodiment 1 are the same as those in Embodiment 1.

Also in Modification 1 of Embodiment 1, the first image 510 and the second image 520 of one lane, and the display region 530 corresponding to the lane are associated with each other by the same color. Accordingly, as in Embodiment 1, the operator can intuitively understand the correspondence relationship between the first image 510 and the second image 520, and the sample ID of each lane, by referring to the color.

Instead of providing a color to the rectangular regions surrounding the first image 510, the second image 520, and the display region 530, a color may be provided to the frame at the outer peripheries of the first image 510, the second image 520, and the display region 530.

### <Modification 2 of Embodiment 1>

In Modification 2 of Embodiment 1, the region to be provided with a color in the screen 500 is changed when compared with Embodiment 1. As shown in FIG. 19(b), in Modification 2 of Embodiment 1, only a lower half region of each of the first image 510 and the second image 520 is provided with a color. The color provided to the lower half region of each of the first image 510 and the second image 520 corresponding to each lane is the same as that in Embodiment 1. In addition, only a lower half region of each display region 530 is provided with a color. The color provided to the lower half region of the display region 530 corresponding to each lane is the same as that in Embodiment 1. The other configurations in Modification 2 of Embodiment 1 are the same as those in Embodiment 1. Also in Modification 2 of Embodiment 1, effects similar to those in Modification 1 of Embodiment 1 are exhibited.

### <Modification 3 of Embodiment 1>

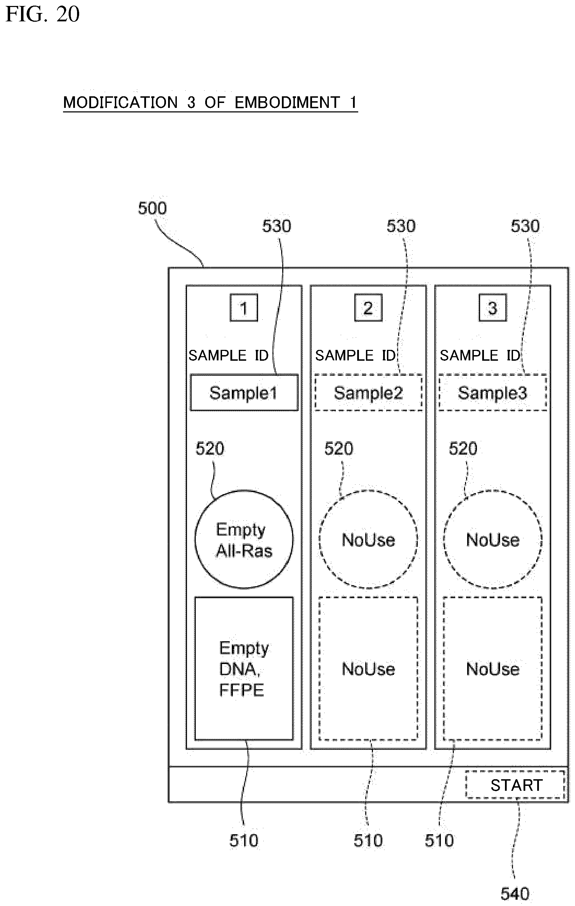

In Modification 3 of Embodiment 1, the screen 500 has a shape that is long in the screen-vertical direction when compared with Embodiment 1. As shown in FIG. 20, in Modification 3 of Embodiment 1, the display region 530 corresponding to the first lane is positioned above the first image 510 and the second image 520 corresponding to the first lane. Similarly, the display region 530 corresponding to the second lane is positioned above the first image 510 and the second image 520 corresponding to the second lane, and the display region 530 corresponding to the third lane is positioned above the first image 510 and the second image 520 corresponding to the third lane. In Modification 3 of Embodiment 1, the first image 510, the second image 520, and the display region 530 are not provided with a color. The other configurations in Modification 3 of Embodiment 1 are the same as those in Embodiment 1.

According to Modification 3 of Embodiment 1, the first image 510, the second image 520, and the display region 530 that correspond to the same lane are arranged in the screen-vertical direction, and thus, even though colors are not provided as in Embodiment 1, the operator can intuitively understand the correspondence relationship. However, in Modification 3 of Embodiment 1, colors may be provided as in Embodiment 1. Then, the operator can further intuitively understand the correspondence relationship.

### <Embodiment 2>

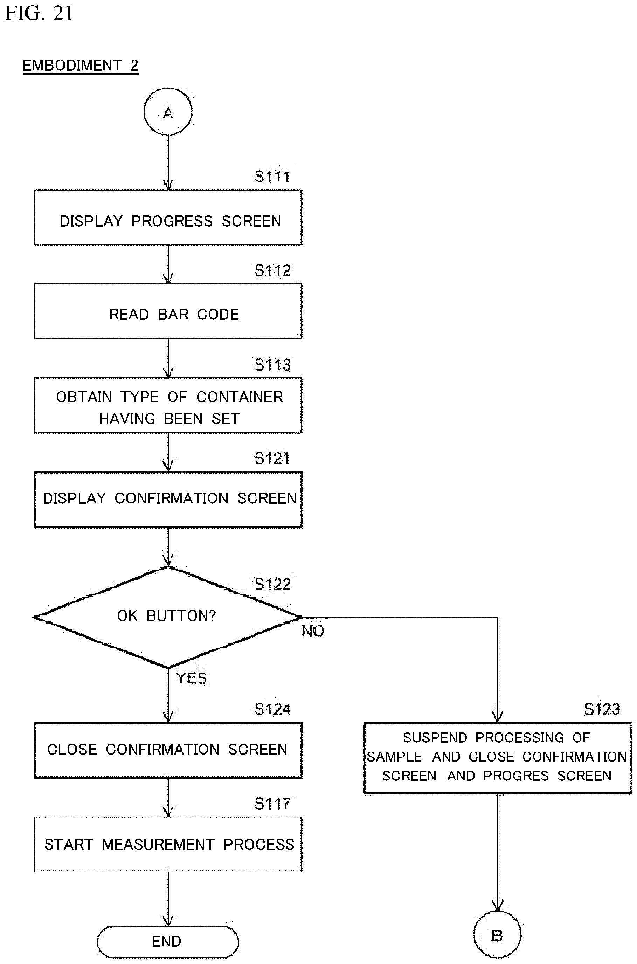

In Embodiment 2, the flow chart shown in FIG. 11 and the screen displayed on the display unit 402 are different when compared with Embodiment 1. In Embodiment 2, as shown in FIG. 21, with respect to the flow chart shown in FIG. 11, step S121 is added after step S113, steps S122 and S123 are added in place of steps S114, S115, step S116 is omitted, and step S124 is added before step S117. The other configurations in Embodiment 2 are the same as those in Embodiment 1.

In the following, steps that are different from those in the flow chart shown in FIG. 11, and a confirmation screen are described.

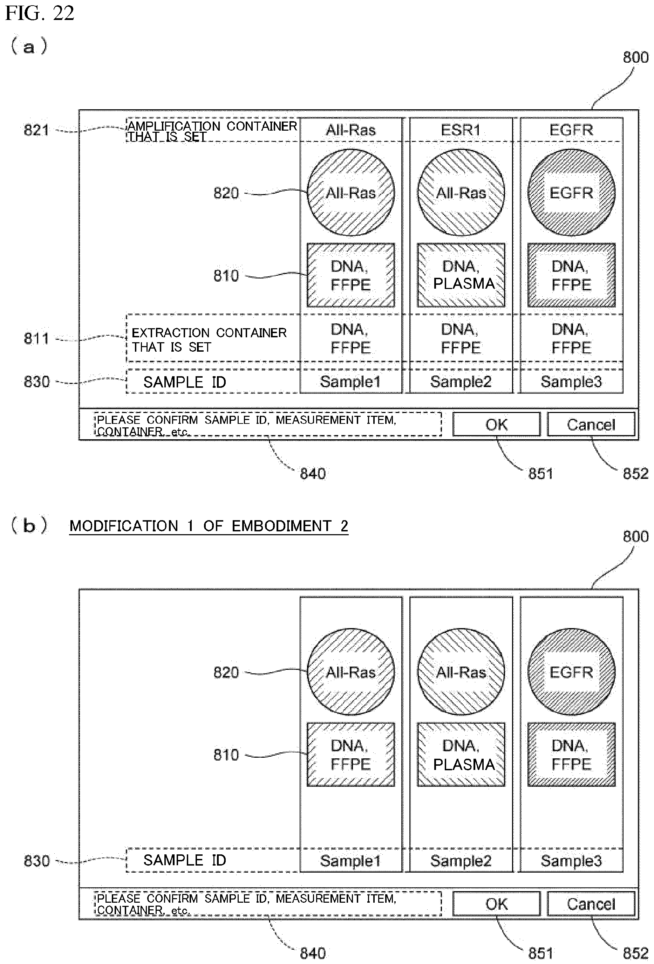

As shown in FIG. 21, in step S121, the controller 401 causes a confirmation screen 800 shown in FIG. 22(a) to be displayed, over the progress screen 700, in the display region of the display unit 402. As shown in FIG. 22(a), the confirmation screen 800 includes: three first images 810 corresponding to the respective lanes; three second images 820 corresponding to the respective lanes; regions 811, 821, 830; a message region 840; an OK button 851; and a cancel button 852.

Similar to the first images 510 on the screen 500, each first image 810 is provided with a color that corresponds to the lane, and displays the type of the extraction container 10 obtained on the basis of a measurement order. Similar to the second images 520 on the screen 500, each second image 820 is provided with a color that corresponds to the lane, and displays the type of the amplification container 20 obtained on the basis of the measurement order. The first image 810 and the second image 820 are disposed in the same manner as that for the first image 510 and the second image 520 on the screen 500. The region 811 is for displaying the types of the extraction containers 10 obtained on the basis of the bar codes of the extraction containers 10 set in the respective lanes. The region 821 is for displaying the types of the amplification containers 20 obtained on the basis of the bar codes of the amplification containers 20 actually set to the respective lanes. The region 830 is for displaying the sample IDs corresponding to the respective lanes. The message region 840 is for displaying a message that urges the operator to confirm the displayed content on the confirmation screen 800. In the message region 840, "Please confirm sample IDs, measurement items, containers, etc." is displayed, for example.

In this manner, information that allows evaluation of whether or not the type of the extraction container 10 set to the extraction container setting part 110 is appropriate for the measurement order is displayed in the first image 810, and the regions 811, 830. Information that allows evaluation of whether or not the type of the amplification container 20 set to the amplification container setting part 120 is appropriate for the measurement order is displayed in the second image 820, and the regions 821, 830.

Subsequently, in step S122, the controller 401 determines whether or not the OK button 851 has been touched. When the operator has determined that appropriate containers are not set, the operator touches the cancel button 852. Accordingly, the controller 401 advances the process from step S122 to step S123, stops the processing of the samples in step S123, and closes the confirmation screen 800 and the progress screen 700. Meanwhile, when the operator has confirmed the displayed content on the confirmation screen 800 and has determined that appropriate containers are set, the operator touches the OK button 851. Accordingly, the controller 401 advances the process from step S122 to S124, and closes the confirmation screen 800 in step S124.

According to Embodiment 2, by referring to the displayed content on the confirmation screen 800, the operator can confirm whether or not an extraction container 10 of an appropriate type is set in each extraction container setting part 110, and whether or not an amplification container 20 of an appropriate type is set in each amplification container setting part 120. Accordingly, the operator can set an extraction container 10 and an amplification container 20 of appropriate types, to the extraction container setting part 110 and the amplification container setting part 120. Thus, appropriate nucleic acid analysis can be performed on the basis of the appropriate containers.

In the confirmation screen 800, when the displayed content in the first image 810 and the displayed content in the region 811 do not match each other, and when the displayed content in the second image 820 and the displayed content in the region 821 do not match each other, an indication that the types of the containers do not match each other, i.e., that containers of wrong types are set, may be displayed on the confirmation screen 800. That the containers of wrong types are set can be displayed by means of, for example, colors of the frames of the first image 810 and the second image 820, an icon for providing an alert, or the like.

### <Modification 1 of Embodiment 2>

As shown in FIG. 22(b), the regions 811, 821 are omitted in the confirmation screen 800 in Modification 1 of Embodiment 2, when compared with the confirmation screen 800 in FIG. 22(a). In Modification 1 of Embodiment 2, since the regions 811, 821 are omitted, there is no need to obtain the types of the containers from the bar codes of the containers having been set. Therefore, in Modification 1 of Embodiment 2, the processes of steps S112, S113 are omitted from the flow chart shown in FIG. 21. The other configurations in Modification 1 of Embodiment 2 are the same as those in Embodiment 2.

Also in Modification 1 of Embodiment 2, the operator can confirm the types of the extraction container 10 and the amplification container 20 that should be set. Thus, the operator can set the extraction container 10 and the amplification container 20 of appropriate types, to the extraction container setting part 110 and the amplification container setting part 120. Therefore, appropriate nucleic acid analysis can be performed on the basis of the appropriate containers.

### <Modification 2 of Embodiment 2>

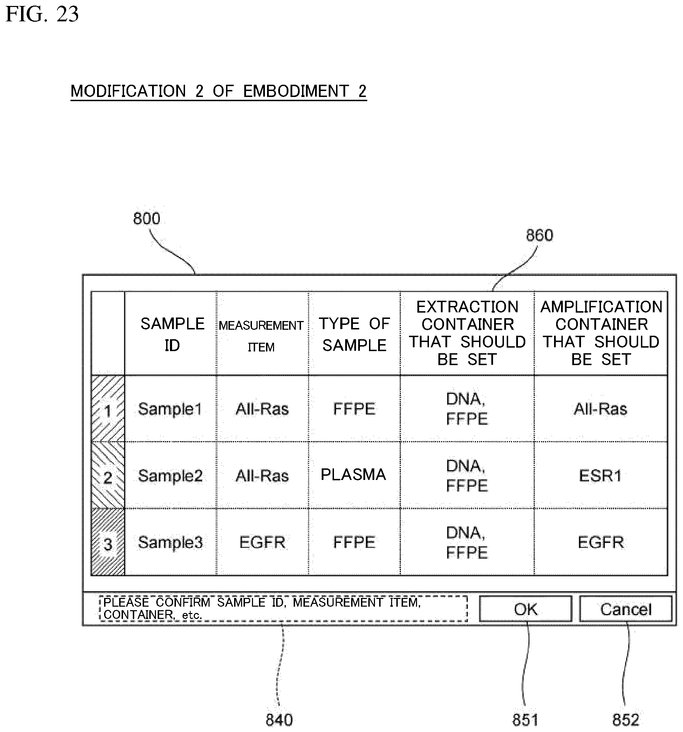

As shown in FIG. 23, the confirmation screen 800 in Modification 2 of Embodiment 2 includes a list region 860 in place of the first images 810, the second images 820, and the regions 811, 821, 830, when compared with the confirmation screen 800 in FIG. 22(a). For each lane, the list region 860 includes, as the items, a sample ID, a measurement item, the type of the sample, the type of the extraction container 10 that should be set, and the type of the amplification container 20 that should be set. In the list region 860, the type of the extraction container 10 that should be set to the extraction container setting part 110, and the type of the amplification container 20 that should be set to the amplification container setting part 120 are displayed in association with the sample ID. The other configurations in Modification 2 of Embodiment 2 are the same as those in Embodiment 2.

According to Modification 2 of Embodiment 2, by referring to the displayed content on the confirmation screen 800, the operator can confirm whether or not the types of the containers having been set and the sample indicated by the sample ID are in an appropriate correspondence relationship. Accordingly, the operator can set containers of appropriate types to the respective container setting parts.

Further, the type of the extraction container 10 obtained from the bar code of the extraction container 10 actually set, and the type of the amplification container 20 obtained from the bar code of the amplification container 20 actually set may be displayed in the list region 860. Then, the operator can further smoothly determine whether or not the containers set to the container setting parts are appropriate.

### <Embodiment 3>

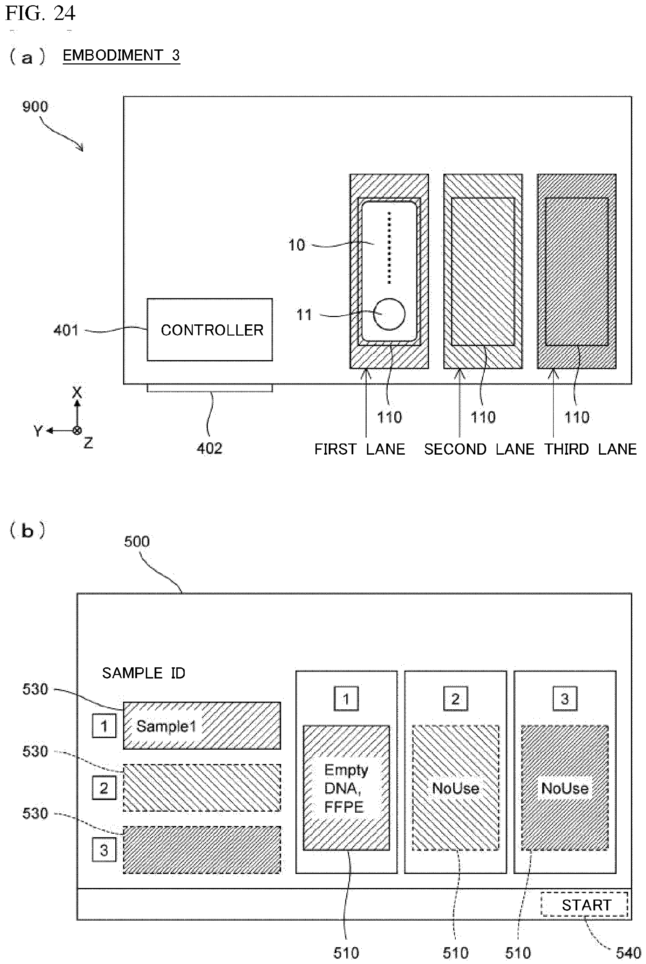

A nucleic acid extraction device 900 of Embodiment 3 is a device for purifying an extract from a sample by using the extraction container 10. As shown in FIG. 24(a), the amplification container setting parts 120 and the detection unit 240 are omitted in the nucleic acid extraction device 900 of Embodiment 3, when compared with the configuration in Embodiment 1 shown in FIG. 1(a). In addition, as shown in FIG. 24(b), the second images 520 are omitted in the screen 500 of Embodiment 3, when compared with the configuration in Embodiment 1 shown in FIG. 1(b). The other configurations in Embodiment 3 are the same as those in Embodiment 1 shown in FIG. 1(a).

In Embodiment 3, a sample is stored in the reaction part 11 of the extraction container 10 and the process is started, as in Embodiment 1, and then an extract is purified in the extraction container 10, as in Embodiment 1. In Embodiment 3, at the time when the extract has been purified in the extraction container 10, the process performed by the nucleic acid extraction device 900 ends.

Specifically, in the process shown in FIG. 10, as in Embodiment 1, when a sample ID has been inputted, the type of the extraction container 10 that should be set to the extraction container setting part 110 is obtained on the basis of the measurement order. Then, in step S106, as shown in FIG. 24(b), the type of the extraction container 10 that should be set is displayed in the first image 510. In the process shown in FIG. 11, when the bar code has been read, and the type of the extraction container 10 actually set to the extraction container setting part 110 has been obtained, it is determined, in step S114, whether or not the type of the extraction container 10 set in the extraction container setting part 110 is appropriate. Then, upon start of a measurement process, only step S201 is performed in the process shown in FIG. 18. Then, the process of purifying an extract from the sample ends.

Also in Embodiment 3, the screen 500 is displayed as in Embodiment 1. Thus, the first relevant information regarding setting of the extraction container 10 to the extraction container setting part 110 plays the role of a guide for the operator to set the extraction container 10 to the extraction container setting part 110. Therefore, by referring to the first relevant information, the operator can appropriately set the extraction container 10 to the extraction container setting part 110. When the first relevant information is displayed, even an operator who is not familiar with the nucleic acid extraction device 900 can appropriately and smoothly set the extraction container 10.

The nucleic acid analysis device 100 of Embodiment 1 may perform only the process of purifying an extract from a sample by using the extraction container 10. In this case, the operator inputs an instruction to perform only the process of purifying an extract, through a menu or the like displayed on the display unit 402. Then, the operator sets an extraction container 10 to the extraction container setting part 110, and touches the start button 540 on the screen 500, to start the process.

### <Embodiment 4>

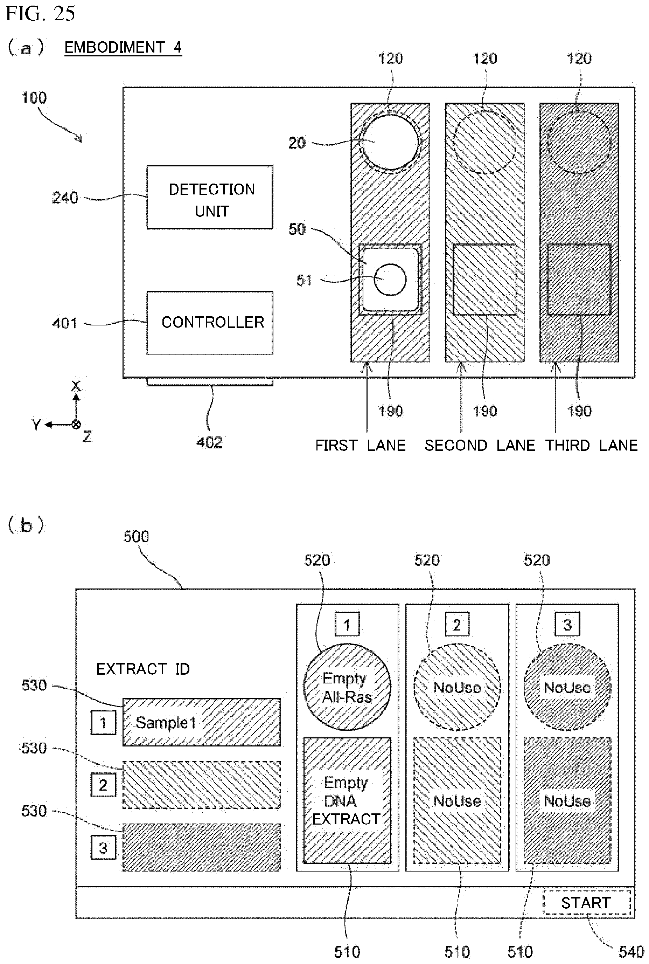

The nucleic acid analysis device 100 of Embodiment 4 is a device for amplifying and detecting nucleic acid in an extract, by using the amplification container 20. As shown in FIG. 25(a), when compared with the configuration of Embodiment 1 shown in FIG. 1(a), the nucleic acid analysis device 100 of Embodiment 4 is provided with extract container setting parts 190 instead of the extraction container setting parts 110, and an extract container 50 is set to each extract container setting part 190. The extract container 50 has formed therein a storage part 51 for storing an extract. As shown in FIG. 25(b), when compared with the configuration of Embodiment 1 shown in FIG. 1(b), "extract ID" is displayed, on the screen 500 of Embodiment 3, in a region above the display regions 530 so as to indicate that each display region 530 is for displaying an extract ID. The other configurations in Embodiment 4 are the same as those shown in FIG. 1(a) of Embodiment 1.

In Embodiment 4, the process is started with an extract being stored in the storage part 51 of the extract container 50, and nucleic acid in the extract is amplified and detected. In Embodiment 4, the process of purifying an extract as in Embodiment 1 is omitted. When amplification, detection, and analysis of nucleic acid in the extract have ended, the process performed by the nucleic acid analysis device 100 ends.

Specifically, in the process shown in FIG. 10, when an extract ID instead of a sample ID has been inputted, the type of the amplification container 20 that should be set to the amplification container setting part 120 is obtained on the basis of the measurement order. In this case, an extract ID, a measurement item, and the type of nucleic acid are registered in the measurement order. Then, in step S106, as shown in FIG. 25(b), the type of the amplification container 20 that should be set is displayed in the second image 520. At this time, the type of nucleic acid in the extract obtained on the basis of the measurement order is displayed in the first image 510. In the process shown in FIG. 11, when the bar code has been read, and the type of the amplification container 20 actually set in the amplification container setting part 120 has been obtained, it is determined, in step S114, whether or not the type of the amplification container 20 set in the amplification container setting part 120 is appropriate. Then, upon start of a measurement process, in the process shown in FIG. 18, the processes of step S202 and thereafter are performed. Then, amplification, detection, and analysis of the nucleic acid in the extract end.

Also in Embodiment 4, the screen 500 is displayed as in Embodiment 1. Thus, the second relevant information regarding setting of the amplification container 20 to the amplification container setting part 120 plays the role of a guide for the operator to set the amplification container 20 to the amplification container setting part 120. Therefore, by referring to the second relevant information, the operator can appropriately set the amplification container 20 to the amplification container setting part 120. When the second relevant information is displayed, even an operator who is not familiar with the nucleic acid analysis device 100 can appropriately and smoothly set the amplification container 20.

The nucleic acid analysis device 100 of Embodiment 1 may perform only the processes of amplification, detection, and analysis of the nucleic acid in the extract, by using the amplification container 20. In this case, the operator inputs an instruction to perform only the processes of amplification, detection, and analysis of the nucleic acid in the extract, through a menu or the like displayed on the display unit 402. Then, the operator sets an amplification container 20 to the amplification container setting part 120, sets an extraction container 10 storing only an extract, to the extraction container setting part 110, and touches the start button 540 on the screen 500, to start the process.

### DESCRIPTION OF THE REFERENCE CHARACTERS

- 10: extraction container
- 20: amplification container
- 100: nucleic acid analysis device
- 110: extraction container setting part
- 112a: first sensor
- 120: amplification container setting part
- 122: second sensor
- 142: reading unit
- 401: controller
- 402: display unit
- 510: first image
- 511: first operation portion
- 520: second image
- 521: second operation portion
- 530: display region
- 810: first image
- 820: second image
- 900: nucleic acid extraction device

## Claims

1. A nucleic acid analysis device comprising:
a plurality of amplification container setting parts each configured to have an amplification container set thereto, the amplification container being configured to have injected therein an extract that contains nucleic acid, the amplification container storing a reagent for amplifying the nucleic acid in the extract;
a display unit; and
a controller configured to cause the display unit to display a screen in which a location of each of the plurality of amplification container setting parts is associated with relevant information regarding setting of the amplification container to the amplification container setting part.

2. The nucleic acid analysis device according to claim 1, wherein
the relevant information includes information that links each of a plurality of samples to a location of an amplification container setting part to which an amplification container storing a reagent for processing the sample is set.

3. The nucleic acid analysis device according to claim 2, wherein
the relevant information includes information that links identification information of each of a plurality of samples to a location of an amplification container setting part to which an amplification container storing a reagent for processing the sample is set.

4. The nucleic acid analysis device according to claim 3, wherein
the relevant information includes a color provided to a display of identification information of each sample and to a location of an amplification container setting part corresponding to the sample.

5. The nucleic acid analysis device according to any one of claims 1 to 4, wherein
the relevant information includes information regarding the type of the amplification container that should be set to each amplification container setting part.

6. The nucleic acid analysis device according to claim 5, wherein
the information regarding the type of the amplification container includes information regarding a measurement item of a sample to be processed.

7. The nucleic acid analysis device according to any one of claims 1 to 6, wherein
when an amplification container of a type corresponding to a sample to be processed is not set to an amplification container setting part, the controller causes the display unit to display, as the relevant information, information indicating that the type of the amplification container is not appropriate.

8. The nucleic acid analysis device according to claim 7, wherein
the amplification container holds information that can specify the type of the amplification container,
the nucleic acid analysis device includes a reading unit configured to read information regarding the type of the amplification container, from the amplification container set to the amplification container setting part, and
when the type of the amplification container corresponding to a sample to be processed does not match the information read by the reading unit, the controller causes the display unit to display, as the relevant information, information indicating that the type of the amplification container set to the amplification container setting part is not appropriate.

9. The nucleic acid analysis device according to claim 7 or 8, wherein
when the amplification container of the type corresponding to the sample to be processed is not set to the amplification container setting part, the controller suspends a process on the sample.

10. The nucleic acid analysis device according to any one of claims 1 to 9, comprising
a sensor configured to detect whether or not the amplification container is set to the amplification container setting part, wherein
on the basis of a detection result by the sensor, the controller causes the display unit to display, as the relevant information, information regarding whether or not the amplification container is set to the amplification container setting part.

11. The nucleic acid analysis device according to any one of claims 1 to 10, wherein
the relevant information includes information that allows evaluation of whether or not the type of the amplification container set to the amplification container setting part is appropriate for a sample to be processed.

12. The nucleic acid analysis device according to any one of claims 1 to 11, wherein
the controller causes the display unit to display the screen in which an image corresponding to the location of each of the plurality of amplification container setting parts is associated with the relevant information.

13. The nucleic acid analysis device according to claim 12, wherein
when a predetermined operation has been performed on the image, the controller causes the display unit to display an operation portion for receiving a measurement item of a sample to be processed.

14. The nucleic acid analysis device according to any one of claims 1 to 13, further comprising
a plurality of extraction container setting parts each configured to have an extraction container set thereto, the extraction container storing a reagent for extracting nucleic acid from a sample, the extraction container being configured to purify an extract containing the nucleic acid by use of the reagent, wherein
the controller causes the display unit to display the screen in which a location of each of the plurality of extraction container setting parts is associated with relevant information regarding setting of the extraction container to the extraction container setting part.

15. The nucleic acid analysis device according to claim 14, wherein
the relevant information includes information regarding the type of the extraction container that should be set to the extraction container setting part.

16. The nucleic acid analysis device according to claim 15, wherein
the information regarding the type of the extraction container includes information regarding the type of a sample to be processed.

17. The nucleic acid analysis device according to any one of claims 14 to 16, wherein
the controller causes the display unit to display the screen in which an image corresponding to the location of each of the plurality of extraction container setting parts is associated with the relevant information.

18. The nucleic acid analysis device according to claim 17, wherein
when a predetermined operation has been performed on the image, the controller causes the display unit to display an operation portion for receiving the type of a sample to be processed.

19. The nucleic acid analysis device according to any one of claims 14 to 18, wherein
an amplification container setting part and an extraction container setting part that belong to the same combination are disposed so as to be arranged in a depth direction of a device body,
a longitudinal direction of the amplification container setting part and the extraction container setting part that belong to the same combination is parallel to the depth direction of the device body, and
amplification container setting parts and extraction container setting parts that belong to different combinations are disposed so as to be arranged in a width direction of the device body.

20. The nucleic acid analysis device according to claim 19, wherein
the extraction container setting parts and the amplification container setting parts are open such that the extraction containers and the amplification containers are allowed to be set from a front side of the device body,
the display unit is disposed at a front face of the device body, and
the controller causes the display unit to display the screen in which: images respectively corresponding to an extraction container setting part and an amplification container setting part of one combination are arranged in a screen-vertical direction; and images respectively corresponding to an extraction container setting part and an amplification container setting part of another combination are arranged in a screen-horizontal direction with respect to the images of the one combination.

21. The nucleic acid analysis device according to claim 19 or 20, wherein
the controller causes the display unit to display the screen in which: an image corresponding to the extraction container setting part and an image corresponding to the amplification container setting part are arranged in a screen-vertical direction; and a region in which identification information of a sample corresponding to an extraction container setting part and an amplification container setting part of each combination is displayed extends in a screen-horizontal direction.

22. A nucleic acid extraction device comprising:
a plurality of extraction container setting parts each configured to have an extraction container set thereto, the extraction container storing a reagent for extracting nucleic acid from a sample, the extraction container being configured to purify an extract containing the nucleic acid by use of the reagent;
a display unit; and
a controller configured to cause the display unit to display a screen in which a location of each of the plurality of extraction container setting parts is associated with relevant information regarding setting of the extraction container to the extraction container setting part.

23. A nucleic acid analysis device comprising:
an extraction container setting part configured to have an extraction container set thereto, the extraction container storing a reagent for extracting nucleic acid from a sample, the extraction container being configured to purify an extract containing the nucleic acid by use of the reagent;
an amplification container setting part configured to have an amplification container set thereto, the amplification container being configured to have injected therein the extract purified in the extraction container, the amplification container storing a reagent for amplifying the nucleic acid in the extract;
a display unit; and
a controller configured to cause the display unit to display a screen in which a location of the extraction container setting part is associated with first relevant information regarding setting of the extraction container to the extraction container setting part, and a location of the amplification container setting part is associated with second relevant information regarding setting of the amplification container to the amplification container setting part.
